(19) Europäisches Patentamt
European Patent Office
Office européen des brevets

(11) Veröffentlichungsnummer: **0 005 507**
**A1**

# (12) EUROPÄISCHE PATENTANMELDUNG

(21) Anmeldenummer: 79101415.2

(22) Anmeldetag: 09.05.79

(51) Int. Cl.²: C 07 D 205/08
C 07 C 121/75
//C07C125/06

(30) Priorität: 18.05.78 CH 5393/78

(43) Veröffentlichungstag der Anmeldung: 28.11.79 Patentblatt 79/24

(84) Benannte Vertragsstaaten: CH DE FR GB IT NL

(71) Anmelder: CIBA-GEIGY AG
Patentabteilung Postfach
CH-4002 Basel(CH)

(72) Erfinder: Schaffner, Karl, Dr.
Im Goldbrunnen 37
CH-4104 Oberwil(CH)

(72) Erfinder: Wehrli, Hansuli, Dr.
Binningerstrasse 41
CH-4153 Reinach(CH)

(72) Erfinder: Müller, Beat, Dr.
Stockackerstrasse 3
CH-4153 Reinach(CH)

(72) Erfinder: Scartazzini, Riccardo, Dr.
Conrad Ferdinand Meyer-Strasse 38
CH-4059 Basel(CH)

## (54) Fragmentierungsverfahren von 3-(2-Hydroxyimino-acetylamino)-2-oxo-azetidin-Verbindungen und die erhaltenen Verbindungen.

(57) Bei der Behandlung von 3-(2-Hydroxyiminoacetylamino)-2-oxo-azetidinverbindungen mit reaktionsfähigen Derivaten von Kohlensäurehalbestern in wässrigen Medien in Gegenwart von Basen tritt eine Fragmentierung ein, die neben 3-Amino-2-oxo-azetidinen auch Nitrile liefert, die gegenüber der 2-Hydroxyiminoacetylgruppe ein Kohlenstoffatom weniger besitzen. Die entstehenden Produkte sind Zwischenprodukte zur Herstellung Nocardicinähnlicher Antibiotika.

Das vorliegende Desacylierungsverfahren bietet gegenüber dem bisher bekannten mehrstufigen Desacylierungsverfahren Vorteile, insofern höhere Ausbeuten an 3-Amino-2-oxoazetidinverbindungen erhalten werden und die Acylgruppe in Form eines um ein Kohlenstoffatom verkürzten Nitriles gefasst werden kann.

EP 0 005 507 A1

CIBA-GEIGY AG
Basel (Schweiz)

4-11709/+

BEZEICHNUNG GEÄNDERT
siehe Titelseite

Fragmentierungsverfahren

Die Erfindung betrifft ein Methodikverfahren zur Herstellung von primären Aminen und/oder Nitrilen, gegebenenfalls in geschützter Form, durch Aufspaltung der Amidbindung von 2-Hydroxyiminoacetamiden mittels einer neuartigen Fragmentierungsreaktion. Die erfindungsgemäss herstellbaren neuen Amine und Nitrile sind, gegebenenfalls in geschützter Form, ebenfalls Gegenstand der Erfindung.

Die Erfindung ist insbesondere von Bedeutung für die Herstellung von 3-Amino-2-oxoazetidinverbindungen, die wertvolle Ausgangsstoffe zur Herstellung entsprechender antibiotisch wirksamer 3-Acylamino-2-oxo-1-azetidinalkansäurederivate sind. Solche 3-Amino- und 3-Acylamino-2-oxo-1-azetidinalkansäurederivate, sowie Verfahren zu ihrer Herstellung, sind beispielsweise aus den Deutschen Offenlegungsschriften 2.529.941, und 2.714.628, und dem Belgischen Patent 849.445 bekannt.

Aus der Deutschen Offenlegungsschrift 2.529.941 ist bekannt, dass die 2-Hydroxyiminoacetamidverbindung Nocardicin-A (gemäss Chemical Abstracts als (3S)-3-[[[4-((3R)-3-Amino-3-carboxypropoxy)phenyl](Z-hydroxyimino) acetyl] amino]-(αR)-α-(4-hydroxyphenyl)-2-oxo-1-azetidinessigsäure zu benennen) in einem mehrstufigen Verfahren zum entsprechenden primären Amin, der (3S)-3-Amino-(αR)-α-(4-hydroxyphenyl)-2-oxo-1-azetidinessigsäure(3-Amino-lactacillansäure), desacyliert werden kann. Dieses Desacylierungsverfahren beruht auf der Reduktion der 2-Hydroxyiminogruppe zur 2-Aminogruppe, Ueberführung derselben in eine Phenyl- oder Naphthylthioureidogruppe und anschliessender Abspaltung der 2-Thioureidoacetylgruppe durch alkalische oder saure Solvolyse. Alternativ kann die 2-Aminogruppe in eine 2-Nitro-4-methoxyanilinogruppe übergeführt werden, worauf die entsprechende 2-Anilinoacetylgruppe durch katalytische Druckhydrierung in Gegenwart von wässrigem Methanol abgespalten wird. In beiden Verfahren sind die Ausbeuten relativ niedrig und ausserdem wird jeweils nur das primäre Amin, d.h. die 3-Amino-lactacillansäure, isoliert. Die ebenfalls wertvolle Seitenkette, die 4-(3-Amino-3-carboxypropoxy)phenylessigsäure oder irgendein Abkömmling davon geht verloren.

Der vorliegenden Erfindung liegt die Aufgabe zugrunde, aus 3-(2-Hydroxyimino-acetyl)amino-2-oxoazetidinverbindungen die 2-Hydroxyiminoacetylgruppe abzuspalten, die 3-Amino-2-oxoazetidinverbindungen unter Erhaltung gegebenenfalls vorhandener optisch aktiver Kohlenstoffatome in hoher Ausbeute und Reinheit herzustellen, und gewünschtenfalls die 2-Hydroxyiminoacetylseitenkette in Form eines entsprechenden, um ein Kohlenstoffatom verringerten Nitrils ebenfalls zu isolieren.

Diese Aufgabe wird durch das erfindungsgemässe, überraschende Fragmentierungsverfahren in gegenüber den bekannten Verfahren überlegener Weise gelöst. Die Ueberlegenheit des erfindungsgemässen Verfahrens liegt in der höheren Ausbeute, in der Möglichkeit der Isolierung der genannten Nitrile und darin, dass das Verfahren als sogenanntes Eintopfverfahren durchgeführt werden kann.

Die Erfindung betrifft demgemäss ein Methodikverfahren zur Herstellung von 3-Amino-2-oxoazetidinverbindungen der Formel

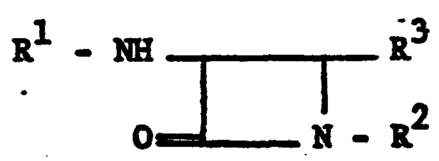

(I) ,

worin $R^1$ Wasserstoff oder eine verätherte Hydroxycarbonylgruppe der Formel $R_a^1$-O-C(=O)-, worin $R_a^1$ ein gegebenenfalls substituierter Kohlenwasserstoffrest ist, $R^2$ Wasserstoff oder einen gegebenenfalls substituierten Kohlenwasserstoffrest und $R^3$ Wasserstoff oder einen gegebenenfalls substituierten Kohlenwasserstoffrest bedeuten, worin funktionelle Gruppen gegebenenfalls geschützt sind, oder Salzen von solchen Verbindungen mit salzbildenden Gruppen, und/oder von Nitrilen der Formel $R^o$-CN, worin $R^o$ ein gegebenenfalls substituierter Phenyl- oder Heterocyclylrest ist, oder Salzen von solchen Verbindungen mit salzbildender Gruppe, dadurch gekennzeichnet, dass man eine 3-(2-Hydroxyiminoacetylamino)-2-oxoazetidinverbindung der Formel

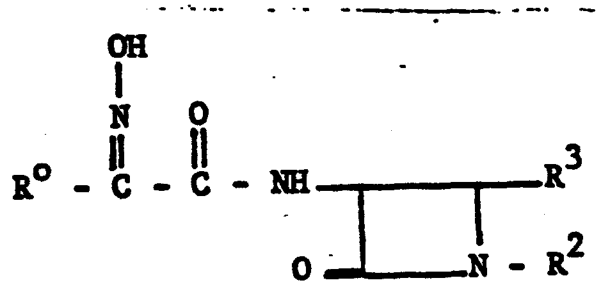

(II) ,

worin $R^o$, $R^2$ und $R^3$ die oben angegebenen Bedeutungen haben, in welchen Resten funktionelle Gruppen gegebenenfalls geschützt sind, oder ein Salz einer solchen Verbindung mit salzbildender Gruppe, in einem wasserhaltigen Medium in Gegenwart einer Base mit einem reaktionsfähigen Derivat eines Kohlensäurehalbesters der Formel $R_a^1$-O-C(=O)-OH, worin $R_a^1$ die oben angegebene Bedeutung hat, behandelt, eine Verbindung der Formel I und/oder ein Nitril $R^o$-CN isoliert und gewünschtenfalls in einer erhaltenen Verbindung gegebenenfalls vorhandene geschützte funktionelle Gruppen durch Abspaltung der Schutzgruppen freisetzt oder gegebenenfalls vorhandene freie funktionellen Gruppen schützt, und gewünschtenfalls eine erhaltene Verbindung mit salzbildender Gruppe in ein Salz oder ein erhaltenes Salz in eine freie Verbindung überführt.

In der vorliegenden Anmeldung enthalten mit "Nieder" bezeichnete organische Reste, wie Niederalkyl, Niederalkoxy, Niederalkanoyl, Niederalkenyl und dergleichen, bis zu 7, bevorzugt bis zu 4 Kohlenstoffatome.

Geschützte funktionelle Gruppen sind auf konventionelle Weise geschützte funktionelle Gruppen, insbesondere geschützte Amino-, Hydroxy-, Mercapto-, Carboxy- und Sulfogruppen. Entsprechende Schutzgruppen sind aus der Peptid-, Penicillin-, Cephalosporin- und Nocardicinchemie bekannt.

In einer Verbindung der Formel I und in dem reaktionsfähigen Derivat des Kohlensäurehalbesters der Formel $R_a^1$-O-C(=O)- ist $R_a^1$ ein gegebenenfalls substituierter aliphatischer, cycloaliphatischer, aromatischer oder araliphatischer Kohlenwasserstoffrest, der beispielsweise bis zu 18 C-Atomen enthält und als Substituenten beispielsweise

Oxo, Niederalkyl, Niederalkoxy, Halogen, Cyan oder Nitro trägt. Typische Reste $R_a^1$ sind Niederalkyl, wie Methyl, Aethyl und insbesondere tert.-Butyl, Oxoniederalkyl, wie Acetonyl, Halogenniederalkyl, wie 2-Brom- oder 2-Jodäthyl oder insbesondere 2,2,2-Trichloräthyl, Cyanniederalkyl, wie 2-Cyanäthyl, Cycloalkyl, wie Cyclohexyl, gegebenenfalls durch Halogen, Niederalkoxy oder Nitro substituiertes Phenyl, wie Phenyl, Pentachlorphenyl, 4-Methoxyphenyl oder 2-Nitrophenyl, und gegebenenfalls im aromatischen Rest durch Halogen, Niederalkoxy oder Nitro substituiertes Benzyl oder Diphenylmethyl, wie Benzyl, 4-Methoxybenzyl, 4-Nitrobenzyl, Diphenylmethyl oder 4,4'-Dimethoxydiphenylmethyl.

Der in den Endstoffen der Formel I und den Ausgangsstoffen der Formel II genannte gegebenenfalls substituierte Kohlenwasserstoffrest $R^2$ hat bis zu 18, bevorzugt bis zu 12, und insbesondere 8 Kohlenstoffatome. Hervorzuheben sind die in den genannten Deutschen Offenlegungsschriften und dem Belgischen Patent genannten Reste $R^2$.

So ist $R^2$ beispielsweise eine Gruppe $-CH(R_a^2)(R_b^2)$ oder eine Gruppe $-C(R_c^2)=C(R_d^2)(R_e^2)$, worin $R_a^2$ freies, verestertes, amidiertes oder in Salzform vorliegendes Carboxy oder Carboxyniederalkyl, Cyan, gegebenenfalls substituiertes Amino oder gegebenenfalls substituiertes Hydroxy, $R_b^2$ gegebenenfalls, z.B. durch Niederalkyl, gegebenenfalls substituiertes Hydroxy, gegebenenfalls substituiertes Amino, Nitro oder Halogen, substituiertes Niederalkyl, Niederalkenyl, Cycloalkyl, Aryl, Heterocyclyl, Aralkyl oder Arylthioalkyl, $R_c^2$ freies, verestertes, amidiertes oder in Salzform vorliegendes Carboxyl, $R_d^2$ Wasserstoff oder Niederalkyl, und $R_e^2$ Niederalkyl, gegebenenfalls, z.B.

durch Niederalkyl, gegebenenfalls substituiertes Hydroxy, gegebenenfalls substituiertes Amino, Nitro oder Halogen, substituiertes Aryl, Heterocyclylthioalkyl oder Arylthio bedeutet.

In den Resten $R^2$ ist verestertes Carboxy, auch im veresterten Carboxyniederalkyl, beispielsweise durch einen aliphatischen, araliphatischen, cycloaliphatischen oder aromatischen Alkohol mit bis zu 18 C-Atomen verestert, und ist beispielsweise Niederalkoxycarbonyl, wie Methoxy-, Aethoxy- oder tert.-Butoxycarbonyl, Acyloxymethoxycarbonyl, wie Pivaloyloxymethoxycarbonyl, Halogenniederalkoxycarbonyl, wie 2-Chlor- oder 2,2,2-Trichloräthoxycarbonyl, gegebenenfalls substituiertes Arylniederalkoxycarbonyl, wie Benzyloxycarbonyl, 4-Methoxy- oder 4-Nitrobenzyloxycarbonyl, Diphenylmethoxycarbonyl oder 4,4'-Dimethoxydiphenylmethoxycarbonyl, Cycloalkyloxycarbonyl, wie Cyclohexyloxycarbonyl, gegebenenfalls substituiertes Phenoxycarbonyl, wie Phenoxycarbonyl, 2- oder 4-Nitrophenoxycarbonyl, 4-Methoxyphenyl, Halogenphenoxycarbonyl, wie Pentachlorphenyloxycarbonyl, und dergleichen.

In den Resten $R^2$ ist amidiertes Carboxy, auch im amidierten Carboxyniederalkyl, durch ein primäres oder sekundäres aliphatisches oder aromatisches Amin oder Hydrazin oder durch Triazin amidiert, und ist beispielsweise Aminocarbonyl, Mono- oder Diniederalkylaminocarbonyl, wie Methyl- oder Dimethylaminocarbonyl, Anilinocarbonyl, Hydrazinocarbonyl oder Azidocarbonyl.

Gegebenenfalls substituiertes Amino in den Resten $R^2$ ist beispielsweise Amino, Mono- oder Diniederalkylamino, Acylamino, wie Acetylamino, oder geschütztes Amino, und gegebenenfalls substituiertes Hydroxy ist beispielsweise Hydroxy, Niederalkoxy, Acyloxy, wie Acetoxy oder Carbamoyloxy, oder geschütztes Hydroxy. Geschütztes

Amino und geschütztes Hydroxy sind durch konventionelle Schutzgruppen, beispielsweise durch eine Gruppe $R_1^a$-O-C(=O)- oder durch eine andere leicht abspaltbare Acylgruppe, wie Formyl, 2-Halogenacetyl, z.B. 2-Brom-oder 2,2,2-Trichloracetyl, oder durch eine gegebenenfalls substituierte Benzylgruppe, wie Benzyl, 4-Methoxybenzyl, 2- oder 4-Nitrobenzyl, Diphenylmethyl und dergleichen, geschütztes Amino oder Hydroxy. Geschütztes Amino ist ferner auch Phthalimido.

In Salzform vorliegendes Carboxy in den Resten $R^2$ ist Carboxy, das eines der unten genannten Metall- oder Ammoniumsalze bildet.

In den Resten $R^2$ ist Aryl ein aromatischer Kohlenwasserstoff, wie Phenyl oder Naphthyl, und Heterocyclyl ist ein 3- bis 10-gliedriger monocyclischer oder bicyclischer Heterocyclylrest mit einem oder mehreren Heteroatomen aus der Gruppe Stickstoff, Sauerstoff und Schwefel, welcher mit einem seiner C-Atome gebunden ist, und ist beispielsweise Aziridinyl, Azetidinyl, Pyrrolyl, 2H-Pyrrolyl, Imidazolyl, Pyrazolyl, Pyridyl, Pyrazinyl, Piperidinyl, Piperazinyl, Pyrimidinyl, Pyridazinyl, Triazolyl, Thiazolinyl, Triazinyl, Pyrrolidinyl, Imidazolidinyl, Oxiranyl, Furanyl, Pyranyl, Thienyl, Morpholinyl, Furazanyl, Oxazolyl, Isoxazolyl, Thiazolyl, Thiadiazolyl, Oxadiazolyl, Indolyl, 3H-Indolyl, Isoindolyl, Indolizinyl, 1H-Indazolyl, Purinyl, Benzimidazolyl, Benzotriazolyl, Chinolinyl, Isochinolinyl, Naphthiridinyl, Chinoxalinyl, Chinazolinyl, Benzofuranyl, Chromenyl, Isobenzofuranyl, Benzothiophenyl, Xanthenyl, Benzoxazolyl, Benzisoxazolyl oder Benzothiazolyl, welche Reste wie angegeben substituiert sein können.

$R^2$ ist beispielsweise 1-Carboxyniederalkyl, worin Niederalkyl ein bis vier C-Atome enthält, 1-Carboxy-1-arylmethyl, worin Aryl Naphthyl oder Phenyl ist, und worin der Phenylring gegebenenfalls durch Niederalkyl, wie Methyl, Halogen wie Chlor oder Brom, Nitro, gegebebenfalls substituiertes Amino und/oder gegebenenfalls substituiertes Hydroxy ein- bis dreifach substituiert ist, 1-Carboxyniederalkenyl, 1-Carboxy-ω-phenylniederalkyl, worin Niederalkyl zwei bis vier C-Atome enthält und Phenyl gegebenenfalls, z.B. durch Hydroxy, substituiert ist, 1-Carboxy-ω-heterocyclylniederalkyl, worin Niederalkyl ein bis vier C-Atome enthält und Heterocyclyl beispielsweise Thienyl, Furyl, durch Niederalkyl substituiertes Thiadiazolyl oder Benthiazolyl ist, 1-Aminoniederalkyl, worin Niederalkyl ein bis vier C-Atome enthält und Amino gegebenenfalls substituiert, z.B. geschützt, ist, 1-Hydroxyniederalkyl, worin Niederalkyl ein bis vier C-Atome enthält und Hydroxy gegebenenfalls substituiert ist, 1-Carboxyniederalkenyl, worin Niederalkenyl zwei bis vier C-Atome enthält und gegebenenfalls durch Phenyl oder Phenylthio substituiert ist, in welchen Gruppen die Carboxygruppe gegebenenfalls in veresterter oder amidierter Form, z.B. als Niederalkoxycarbonyl, Benzyloxycarbonyl, Diphenylmethoxycarbonyl und dergleichen oder als Azidocarbonyl, vorliegt.

Hervorzuhebende Reste $R^2$ sind Wasserstoff, α-Carboxy-4-hydroxybenzyl oder α-Carboxy-4-hydroxybenzyl, worin die Carboxygruppe verestert und/oder die 4-Hydroxygruppe geschützt oder anderweitig substituiert ist, wie α-Carbomethoxy-4-methoxybenzyl, α-Carbomethoxy-4-benzyloxybenzyl, α-Carbobenzyloxy-4-benzyloxybenzyl, α-Pivaloyl-

oxymethoxycarbonyl-4-hydroxybenzyl, α-Carbomethoxy-4-acetoxybenzyl, α-Carboxy-4-benzyloxycarbonyloxybenzyl, α-Carboxy-4-tert.-butyloxycarbonyloxybenzyl, α-Carboxy-4-benzoyloxybenzyl, α-Carboxy-4-(2,2,2-trichloracetylaminocarbonyloxy)benzyl, α-Diphenylmethoxycarbonyl-4-hydroxybenzyl, α-Diphenylmethoxycarbonyl-4-tert.-butyloxybenzyl, α-Diphenylmethoxy-4-benzyloxycarbonyloxybenzyl, α-Carboxybenzyl, α-Carbomethoxybenzyl, α-Carbobenzyloxybenzyl, α-Carbomethoxy-3-amino-benzyl, α-Carbomethoxy-3-benzyloxycarbonylaminobenzyl, α-Carbomethoxy-3-nitrobenzyl, α-Carbomethoxy-4-methylbenzyl, α-Carbomethoxy-3,4,5-trimethoxybenzyl, α-Carbomethoxy-4-methylthio-benzyl, α-Carbomethoxy-3-brom-4-hydroxybenzyl, α-Carboxy-3,5-dibrom-4-hydroxybenzyl, α-Carbomethoxy-3,5-dichlor-4-hydroxybenzyl oder α-Carboxy-4-carbamoyloxybenzyl, ferner Carboxymethyl, Carboäthoxymethyl, Carbobenzyloxymethyl, 1-Carboxy-2-methylpropyl, 1-Carbomethoxy-2-methylprop-1-en-1-yl, 1-Carboäthoxy-2-phenylvinyl, 1-Carboxy-2-phenylvinyl, 1-Carboxy-2-phenylthiovinyl, 1-Phenyl-2-carboxyäthyl, 1-Carboxy-2-phenylthioäthyl, 1-Carboxy-2-methoxy-2-phenyläthyl, 1-Carboxy-2-(4-hydroxyphenyl)-äthyl, 1-Azidocarbonyl-2-methylpropyl, 1-(2,2,2-Trichloräthoxycarbonyl-amino)-2-methylpropyl, 1-tert.-Butyloxycarbonylamino-2-methylpropyl, 1-Acetoxy-2-methyl-propyl, 1-Methoxyallyl, Carboxy-2-thienylmethyl, Carboxy-2-furylmethyl, Carboxy-1-naphthyl-methyl, 1-Carboxy-3-(5-methyl-1,3,4-thiadiazol-2-yl)-propyl und 1-Carboxy-2-methyl-3-benzthiazol-2-yl-propyl.

Die gegebenenfalls substituierten Kohlenwasserstoffreste $R^3$ haben bis zu 18, bevorzugt bis zu 12 und insbesondere bis zu 8 Kohlenstoffatome. Hervorzuheben

sind die in dem Belgischen Patent 849,445 genannten Reste $R^3$, nämlich Wasserstoff, Hydroxymethyl, Aryl, insbesondere substituiertes oder unsubstituiertes Phenyl, oder Aralkenyl, insbesondere substituiertes oder unsubstituiertes 2-Phenylvinyl.

Im Ausgangsmaterial der Formel II und im Nitril der Formel $R^o$-CN ist $R^o$ beispielsweise durch Hydroxy, veräthertes oder verestertes Hydroxy und/oder Halogen substituiertes Phenyl oder durch Hydroxy, veräthertes oder verestertes Hydroxy, Halogen und/oder gegebenenfalls substituiertes Amino substituiertes Heterocyclyl.

$R^o$ ist beispielsweise Hydroxyphenyl, wie 4-Hydroxyphenyl, Niederalkoxyphenyl, wie 4-Methoxyphenyl, Niederalkenyloxyphenyl, wie 4-Allyloxyphenyl, Aralkyloxyphenyl, wie 4-Benzyloxyphenyl, Niederalkoxycarbonylniederalkoxyphenyl, wie 4-Methoxycarbonylmethoxyphenyl, Carboxyniederalkoxyphenyl, wie 4-Carboxymethoxyphenyl, geschütztes Hydroxyphenyl, wie gegebenenfalls substituiertes Niederalkoxycarbonyloxyphenyl, z.B. 4-tert.-Butyloxycarbonyloxyphenyl, 4-(2,2,2-Trichloräthoxycarbonyloxy)-phenyl oder 4-Benzyloxycarbonyloxyphenyl, ω-Carboxy-ω-hydroxyniederalkoxyphenyl, wie 4-(3-Carboxy-3-hydroxypropoxy)phenyl, ω-Carboxy- oder ω-Niederalkoxycarbonylniederalkoxyphenyl, wie 4-(3-Carboxypropoxy)phenyl oder 4-(3-Methoxycarbonylpropoxy)phenyl, ω-Aminoniederalkoxyphenyl, worin Amino gegebenenfalls geschützt ist, wie 4-(3-Aminopropoxy)phenyl, oder 4-(3-tert.-Butyloxycarbonylaminopropoxy)phenyl, oder ω-Carboxy-ω-amino-niederalkoxyphenyl, worin die Carboxy- und die Aminogruppe gegebenen-

falls in substituierter, wie geschützter Form vorliegen. $R^o$ ist insbesondere 4-(3-Carboxy-3-aminopropoxy)phenyl, worin Carboxy gegebenenfalls in veresterter Form, z.B. als Niederalkoxycarbonyl, wie Methoxycarbonyl, als physiologisch spaltbare Estergruppe, wie Niederalkanoyloxymethoxycarbonyl, wie Pivaloyloxymethoxycarbonyl, oder als leicht spaltbare Estergruppe, wie als 2,2,2-Trichloräthoxy-, Benzyloxy-, 4-Methoxybenzyloxy-, 2- oder 4-Nitrophenyloxy- oder Diphenylmethoxycarbonyl, und Amino gegebenenfalls in acylierter Form, z.B. als gegebenenfalls substituiertes Niederalkanoylamino, wie Acetylamino, 2-Chloracetylamino, 2,2,2-Trifluoracetylamino, 2-(4-Chlor-2-nitrophenoxy)-acetylamino, 2-Phenyl-2-sulfoacetylamino, Glycylamino, N-Phthaloylglycylamino, 2-Phenylglycylamino, Phthalimido, Benzoylamino, gegebenenfalls substituiertes Niederalkoxycarbonylamino oder Niederalkoxythiocarbonylamino, wie tert.-Butyloxycarbonylamino, 2-Chloräthoxycarbonylamino, 2,2,2-Trichloräthoxycarbonylamino, 4-Methoxybenzyloxycarbonylamino, Diphenylmethoxycarbonylamino, oder Aethoxythiocarbonylamino, als gegebenenfalls substituiertes Ureido oder Thioureido, wie 3-Phenylureido oder 3-Phenylthioureido, oder als gegebenenfalls substituiertes Niederalkyl- oder Niederalkenylamino, wie Dimethylamino, Isopropylamino, 2-Carbomethoxyäthylamino, 2-Carboxyäthylamino, Sulfomethylamino, 1-Sulfoäthylamino, Tritylamino oder 2-Carbomethoxy-1-methylvinylamino, oder als substituiertes Arylamino, wie 2-Nitro-4-carbomethoxy-anilino, vorliegen, und worin die Phenylgruppe gegebenenfalls durch Halogen, wie Chlor, substituiert ist. $R^o$ ist ferner einer der unter $R^2$ genannten gegebenenfalls substituierten hetero-

cyclischen Reste, wie Furyl, z.B. 2-Furyl, Thienyl, z.B. 2-Thienyl, Thiazolyl, z.B. 2-Amino- oder 2-(2,2,2-Trifluoracetamido)-1,3-thiazol-4-yl.

Ausgangsverbindungen der Formel II können in racemischer oder optisch aktiver Form eingesetzt werden, und die Hydroxyiminogruppe kann in Z- oder E-Stellung vorliegen. Bevorzugt werden solche Ausgangsverbindungen der Formel II eingesetzt, in denen die vorhandenen unsymmetrisch substituierten C-Atome die den entsprechenden C-Atomen des Nocardicins A entsprechenden Konformationen aufweisen.

Die Erfindung ist von besonderer Bedeutung zur Desacylierung fermentativ herstellbarer Verbindungen der Formel II. Dementsprechend werden fermentativ herstellbare Verbindungen der Formel II als Ausgangsmaterialien bevorzugt (siehe Britische Patentschrift 1.388.198 bzw. die ihr entsprechende Deutsche Offenlegungsschrift 2.242.699), insbesondere das bereits genannte Nocardicin A, sowie das Nocardicin B (das E-Isomere zu Nocardicin A), das Nocardicin E (gemäss Chemical Abstracts als (3S)-3-[[(Z-Hydroxyimino)(4-hydroxyphenyl)acetyl]amino]-(αR)-α-(4-hydroxyphenyl)-2-oxo-1-azetidinessigsäure zu bezeichnen) und das Nocardicin F (E-Isomeres von Nocardicin E).

Im Ausgangsmaterial der Formel II können funktionelle Gruppen, ausser der Hydroxyiminogruppe, in freier oder geschützter Form vorliegen. Im Verlaufe des Verfahrens werden acylierbare Gruppen, wie Hydroxy- und Aminogruppen, durch das reaktionsfähige Derivat des Kohlen-

säurehalbesters der Formel $R_a^1$-O-C(=O)-OH acyliert. In den Produkten der Formel I und im Nitril $R^o$-CN vorhandene acylierbare Gruppen liegen daher nach beendeter Reaktion zunächst in durch die Gruppe $R_a^1$-O-C(=O)- acylierter Form vor. Je nach Art der Aufarbeitung können die Acylgruppen $R_a^1$-O-C(=O)- oder andere vorhandene Schutzgruppen abgespalten werden.

Salze von Verbindungen der Formeln I, II oder $R^o$-CN sind insbesondere diejenigen von solchen Verbindungen mit einer sauren Gruppierung, wie einer Carboxy- oder Sulfogruppe, in erster Linie Metall- oder Ammoniumsalze, wie Alkalimetall- und Erdalkalimetall-, z.B. Natrium-, Kalium-, Magnesium- oder Calciumsalze, sowie Ammoniumsalze mit Ammoniak oder geeigneten organischen Aminen, wobei in erster Linie aliphatische, cycloaliphatische, cycloaliphatisch-aliphatische und araliphatische primäre, sekundäre oder tertiäre Mono-, Di- oder Polyamine, sowie heterocyclische Basen für die Salzbildung in Frage kommen, wie Niederalkylamine, z.B. Triäthylamin, Hydroxy-niederalkylamine, z.B. 2-Hydroxyäthylamin, Di-(2-hydroxyäthyl)-amin oder Tri-(2-hydroxyäthyl)-amin, basische aliphatische Ester von Carbonsäuren, z.B 4-Aminobenzoesäure-2-diäthylamino-äthylester, Niederalkylenamine, z.B. 1-Aethyl-piperidin, Cycloalkylamine, z.B. Bicyclohexylamin, oder Benzylamine, z.B. N,N'-Dibenzyläthylendiamin, ferner Basen vom Pyridintyp, z.B. Pyridin, Collidin oder Chinolin. Verbindungen der Formeln I, II oder $R^o$-CN, die eine basische Gruppe aufweisen, können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure oder p-Toluolsulfonsäure, bilden. Verbindungen der Formeln I, II oder $R^o$-CN mit einer sauren und einer basischen Gruppe können auch in Form von inneren Salzen, d.h. in zwitterionischer Form, vorliegen.

Die bei Durchführung des Verfahrens zu verwendenden reaktionsfähigen funktionellen Derivate eines Kohlensäurehalbesters der Formel $R^1_a$-O-C(=O)-OH sind insbesondere Anhydride, gemischte Anhydride, insbesondere mit Halogenwasserstoffsäuren oder der Stickstoffwasserstoffsäure, sowie reaktionsfähige Ester, wie mit Halogenphenolen, z.B. mit 2,4,5-Trichlorphenol , oder mit Hydroxyiminoverbindungen, z.B. mit 2-Hydroxyimino-2-phenylacetonitril. Solche Verbindungen sind aus der Peptid-, Penicillin-, Cephalosporin- und Nocardicinchemie als Reagentien zum Schützen von Amino- und Hydroxygruppen bekannt. Hervorzuheben sind entsprechende Halogenameisensäureester , wie Chlorameisensäureester, z.B. 2,2,2-Trichloräthoxycarbonylchlorid, tert.-Butyloxycarbonylchlorid, Carbobenzoxychlorid, 4-Methoxycarbobenzoxychlorid und 4-Nitrocarbobenzoxychlorid, oder Fluorameisensäureester, z.B. tert.- Butyloxycarbonylfluorid, oder auch Azidoameisensäureester , z.B. tert.-Butyloxycarbonylazid, reaktionsfähige 2,4,5-Trichlorphenylester, z.B. tert.- Butyl-2,4,5-trichlorphenylcarbonat und 4-Methoxybenzyl-2,4,5-trichlorphenylcarbonat, oder reaktionsfähige Hydroxyiminoester, z.B. 2-tert.- Butyloxycarbonyloxyimino-2-phenylacetonitril, und insbesondere symmetrische Anhydride, z.B. das Di-tert.-butyldicarbonat.

Als wasserhaltige Medien, in denen das Verfahren durchgeführt wird, eignen sich irgend welche mit Wasser mischbare, nicht nucleophile, organische Lösungsmittel,

beispielsweise Aether, wie Tetrahydrofuran oder Polyäther, wie verätherte Aethylen- oder Polyäthylenglycole, z.B. Diäthoxyäthan, oder cyclische Polyäthylenoxide, z.B. Dioxan, oder Kronenäther, wie 15-Crown-5 oder 18-Crown-6, Ketone, wie Alkylketone, z.B. Aceton, Amide, wie Diniederalkylamide, z.B. Dimethylformamid, niederalkyliertes Phosphorsäuretriamid, wie Hexametapol, oder Diniederalkylsulfoxide, wie Dimethylsulfoxid, die mindestens ein Aequivalent, im allgemeinen bis etwa 500 Aequivalente (bezogen auf eingesetztes Ausgangsmaterial) Wasser enthalten.

Geeignete Basen sind die bei Acylierungsreaktionen üblichen anorganischen oder organischen Basen, beispielsweise Alkali- oder Erdalkalimetallbasen, wie Hydroxide oder Carbonate von Natrium, Kalium oder Calcium, insbesondere Natriumhydroxid und Natriumcarbonat, sowie organische tertiäre Stickstoffbasen, wie Triniederalkylamine, z.B. Triäthylamin oder Aethyldiisopropylamin, N,N-Diniederalkylaniline, wie N,N-Dimethylanilin, oder cyclische Stickstoffbasen, wie 1,5-Diazabicyclo[5.4.0]undec-5-en oder 1,5-Diazabicyclo[4.3.0]non-5-en.

Die Zugabe des reaktionsfähigen funktionellen Derivates eines Kohlensäurehalbesters der Formel $R_a^1$-O-C(=O)-OH erfolgt bei Raumtemperatur bis etwa 100° C. Falls in dem Ausgangsmaterial der Formel II ausser der Hydroxyiminogruppe noch weitere acylierbare funktionelle Gruppen, wie Amino- oder Hydroxygruppen, vorhanden sind, werden diese durch das verwendete Acylierungsreagens entweder vorrangig oder gleichzeitig mit der zu acylierenden Hydroxyiminogruppe acyliert. Beispielsweise kann eine Aminogruppe vorrangig bei Raumtemperatur acyliert werden. Die Acylierung der Hydroxyiminogruppe kann ebenfalls bei niederer Temperatur vorgenommen werden, während die darauf

folgende Fragmentierung mit Vorteil bei erhöhter Temperatur, d.h. bei 40-80°, bevorzugt bei 50-60°, erfolgt.

Die Menge des zu verwendenden Acylierungsmittels richtet sich nach der Anzahl acylierbarer funktioneller Gruppen im Ausgangsmaterial und danach, ob das bei der Fragmentierung zunächst entstehende Amin der Formel I, worin $R^1$ Wasserstoff ist, vollständig in ein acyliertes Amin der Formel I, worin $R^1$ eine Gruppe $R^1_a$-O-C(=O)- ist, umgewandelt werden soll und ob gegebenenfalls vorhandene Hydroxygruppen, insbesondere phenolische Hydroxygruppen ebenfalls vollständig acyliert werden sollen. Bevorzugt wird soviel Acylierungsmittel zugegeben, dass am Ende der Reaktion sämtliche acylierbaren Gruppen in den Endprodukten durch die Gruppe $R^1_a$-O-C(=O)-acyliert sind. Da das zu verwendende reaktionsfähige funktionelle Derivat des Kohlensäurehalbesters in dem wässerigen Medium im Verlaufe der Reaktion zum Teil hydrolysiert wird, muss ein Ueberschuss davon verwendet werden, der bis zum Ende der Reaktion gegebenenfalls bei niederen Temperaturen, zugefügt wird. Man kann auch die Fragmentierung zunächst mit einem Acylierungsmittel, z.B. Carbobenzoxychlorid, durchführen, und dann gegebenenfalls noch vorhandene phenolische Hydroxygruppen mit einem zweiten Acylierungsmittel, z.B. Di-tert.-Butyldicarbonat, acylieren.

Der Fortgang der Reaktion kann UV-spektrophotometrisch oder auch dünnschichtchromatographisch verfolgt werden.

Die erhaltene Verbindung der Formel I kann von dem gleichzeitig entstehenden Nitril der Formel $R^o$-CN durch übliche Trennungsmethoden, wie Kristallisation, Chromatographie oder Verteilungsoperationen, abgetrennt werden, oder man derivíert ein zunächst erhaltenes Gemisch (z.B. können erhaltene Verbindungen mit Carboxyl-

gruppen in Ester, wie Diphenylmethylester übergeführt werden) und trennt dann die erhaltenen Derivate.

In einer erhaltenen Verbindung der Formel I, in einem erhaltenen Nitril R°-CN oder in einem erhaltenen Gemisch davon, können gegebenenfalls vorhandene freie funktionelle Gruppen, die unter den Fragmentierungsbedingungen nicht acylierbar sind, insbesondere freie saure Gruppen, z.B. freie Carboxylgruppen, auf an sich bekannte Weise, insbesondere durch Veresterung, geschützt werden, wobei die erhaltenen Ester leicht wieder spaltbar sein sollten. Zahlreiche solcher Ester sind bekannt und umfassen insbesondere Niederalkylester, wie Methyl-, Aethyl- oder tert.-Butylester, Halogenniederalkylester wie 2,2,2-Trichloräthyl- oder 2-Jodäthylester, 2-Ketoniederalkylester, wie Acetonylester oder Phenacylester, 2-Cyanniederalkylester, wie 2-Cyanäthylester, gegebenenfalls an der $CH_2$-Gruppe durch Niederalkyl oder Phenyl und/oder im Phenylring durch Nitro, Niederalkoxy oder Halogen substituierte Benzylester, wie Benzyl-, 2- oder 4-Nitrobenzyl-, 2-Methoxybenzyl-, 3,4-Dimethoxybenzyl oder 4-Chlorbenzylester, Diphenylmethyl- oder 4,4'-Dimethoxydiphenylmethylester, oder Tritylester, oder gegebenenfalls durch Nitro oder Halogen substituierte Phenylester, wie 2- oder 4-Nitrophenyl- oder Pentachlorphenylester.

Die Veresterung wird nach den üblichen Methoden, z.B. durch Behandeln mit einem entsprechenden konventionellen Veresterungsmittel, wie einer Halogenidverbindung, z.B. Methyljodid, Aethyljodid, Benzylchlorid, Phenacylbromid und dergleichen; einem entsprechenden Sulfat, wie Dimethylsulfat, Diäthylsulfat und dergleichen, einem entsprechenden Sulfonat, wie Methyl- oder Aethylbenzolsulfonat oder Methyl- oder Aethyl-p-toluolsulfonat,

oder Methyl-fluorsulfonat oder Methyl-trifluormethylsulfonat und dergleichen; einer entsprechenden Diazoverbindung, wie Diazomethan, Diphenyldiazomethan und dergleichen; oder einem entsprechenden Alkohol, wie Methanol, 2,2,2-Trichloräthanol und dergleichen, gegebenenfalls in Gegenwart eines Kondensationsmittels durchgeführt.

In Falle der Veresterung mit einem Halogenid, Sulfat oder Sulfonat wird in Gegenwart einer Base, wie einer der in der Hauptreaktion genannten Basen, gearbeitet oder man setzt die saure Verbindung als Salz, z.B. als Alkalimetallsalz, ein. Wenn ein Alkohol als Veresterungsmittel benutzt wird, gelangen konventionelle Kondensationsmittel, wie ein Carbodiimid, z.B. N,N'-Dicyclohexylcarbodiimid, N-Cyclohexyl-N'-morpholinoäthylcarbodiimid oder N,N'-Diäthylcarbodiimid, zur Anwendung. Die Veresterung erfolgt in einem der üblichen Lösungsmittel bei Raumtemperatur oder auch bei erniedrigter oder erhöhter Temperatur.

In einer erhaltenen Verbindung der Formel I oder in einem erhaltenen Nitril $R^o$-CN mit geschützten funktionellen Gruppen können die Schutzgruppen auf konventionelle Weise abgespalten werden. Falls mehrere geschützte funktionelle Gruppen vorhanden sind, kann die Abspaltung gleichzeitig, selektiv oder nacheinander erfolgen, je nach Art der Schutzgruppe und nach Art der Abspaltungsreaktion.

Beispielsweise kann eine tert.-Butyloxycarbonylamino- oder Diphenylmethoxycarbonylaminogruppe durch Behandeln mit Ameisen-, Trifluoressig- oder auch p-Toluolsulfonsäure, eine 2,2,2-Trichloräthoxycarbonylamino- oder 2-Jodäthoxycarbonylaminogruppe oder auch eine p-Nitrobenzyloxycarbonylaminogruppe durch Behandeln mit geeigneten Reduktionsmitteln, wie Zink in Gegenwart von wässriger Essigsäure bzw. Wasserstoff in Gegenwart eines Palladiumkatalysators, eine tert.-Butyloxycarbonyloxygruppe durch Behandeln mit Ameisen- oder Trifluoressigsäure, oder eine 2,2,2-Trichloräthoxycarbonyloxygruppe oder p-Nitrobenzyloxycarbonyloxygruppe durch Behandeln mit einem chemischen Reduktionsmittel, wie Zink in Gegenwart von wässriger Essigsäure, oder mit Wasserstoff in Gegenwart eines Palladiumkatalysators, gegebenenfalls selektiv, gespalten werden.

Eine durch eine geeignete 2-Halogenniederalkyl-, eine Arylcarbonylmethyl- oder eine 4-Nitrobenzylgruppe veresterte Carboxylgruppe kann z.B. durch Behandeln mit einem chemischen Reduktionsmittel, wie einem Metall, z.B. Zink, oder einem reduzierenden Metallsalz, wie einem Chrom-IIsalz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie Essig-, sowie Ameisensäure, oder eines Alkohols, wobei man vorzugsweise Wasser zugibt, eine durch eine Arylcarbonylmethylgruppe veresterte Carboxylgruppe auch durch Behandeln mit einem nucleophilen, vorzugsweise salzbildenden Reagens, wie Natriumthiophenolat oder Natriumjodid, und eine durch 4-Nitrobenzyl veresterte Carboxylgruppe auch durch Behandeln mit einem Alkalimetall-, z.B. Natriumdithionit, in die freie Carboxylgruppe übergeführt werden. Eine durch eine geeignete Arylmethylgruppierung veresterte Carboxylgruppe kann z.B. durch Bestrahlen, vorzugsweise mit ultraviolettem Licht, z.B. unter 290 mμ, wenn die Arylmethylgruppe z.B. einen gegebenenfalls in 3-, 4- und/oder 5-Stellung, z.B. durch Niederalkoxy- und/oder Nitrogruppen substituierten Benzylrest darstellt, oder mit längerwelligem

ultraviolettem Licht, z.B. über 290 mμ, wenn die Arylmethylgruppe z.B. einen in 2-Stellung durch eine Nitrogruppe substituierten Benzylrest bedeutet, ferner eine durch eine geeignet substituierte Methylgruppe, wie tert.-Butyl oder Diphenylmethyl, veresterte Carboxylgruppe z.B. durch Behandeln mit einem geeigneten sauren Mittel, wie Ameisensäure oder Trifluoressigsäure, gegebenenfalls unter Zugabe einer nucleophilen Verbindung, wie Phenol oder Anisol, sowie eine hydrogenolytisch spaltbare veresterte Carboxylgruppe, z.B. Benzyloxycarbonyl oder 4-Nitrobenzyloxycarbonyl, durch Hydrogenolyse, z.B. durch Behandeln mit Wasserstoff in Gegenwart eines Edelmetall-, z.B. Palladiumkatalysators, gespalten und freigesetzt werden. Eine gegebenenfalls in 2-Stellung durch Niederalkyl oder Aryl substituierte 2-Oxoäthoxycarbonyl- oder 2-Cyanäthoxycarbonylgruppe, z.B. die Acetonyloxycarbonyl- oder 2-Cyanäthoxycarbonylgruppe, kann unter milden Bedingungen, d.h. bei Raumtemperatur oder unter Kühlen, durch Behandeln mit einer geeigneten Base in das entsprechende Salz dieser Carboxylgruppe übergeführt werden, aus dem die freie Carboxylgruppe durch Ansäuern erhältlich ist. Geeignete Basen sind nucleophil reagierende Metall-, wie Erdalkalimetall- und insbesondere Alkalimetallbasen, wie entsprechende Hydroxide, Carbonate, Bicarbonate, Alkoxide, Phenolate, Mercaptide, Thiophenolate oder Amide, z.B. Natriumhydroxid, Natriumcarbonat, Natriumbicarbonat, Natriumäthanolat, Natriumthiophenolat, Natriumamid oder Natrium-morpholid, oder entsprechende Lithium- oder Kaliumverbindungen, die in Wasser, wässrigen oder Hydroxylgruppen enthaltenden oder auch in polaren inerten Lösungsmitteln unter nachträglicher Behandlung mit Wasser zur Anwendung gelangen. Zur Spaltung der 2-Cyanäthoxycarbonylgruppen können auch tertiäre Amine,

wie Triniederalkylamin, z.B. Triäthylamin oder Hünigbase, oder cyclische oder bicyclische Amine oder Imine, wie N-Methylmorpholin oder 1,5-Diazabicyclo[5.4.0]undec-5-en, in einem inerten Lösungsmittel, wie Methylenchlorid oder Tetrahydrofuran, benutzt werden, wobei direkt die entsprechenden Ammoniumsalze der Carboxylverbindung erhalten werden. Eine Pentachlorphenyloxycarbonylgruppe kann unter milden Bedingungen, z.B. durch verdünnte Natriumcarbonat- oder Natriumbicarbonatlösung oder durch eine organische Base in Gegenwart von Wasser, in eine freie Carboxylgruppe übergeführt werden.

Die Abspaltung von Schutzgruppen kann gewünschtenfalls selektiv oder gleichzeitig vorgenommen werden.

So kann eine tert.-Butyloxycarbonylaminogruppe selektiv durch Behandeln mit p-Toluolsulfonsäure in die freie Aminogruppe, bzw. in das enstprechende p-Toluolsulfonsäuresalz davon, übergeführt werden, wobei gleichzeitig vorhandene tert.-Butyloxycarbonyloxygruppen oder Diphenylmethoxycarbonylgruppen nicht gespalten werden. Andererseits kann eine tert.-Butyloxycarbonyloxygruppe durch Behandeln mit Basen, wie wässriger Natriumcarbonatlösung, in die Hydroxygruppe gespalten werden, wobei gleichzeitig vorhandene tert.-Butyloxycarbonylamino- und Diphenylmethoxycarbonylgruppen nicht gespalten werden. Durch Behandeln mit Trifluoressigsäure, gegebenenfalls in Gegenwart von Anisol, können die tert.-Butyloxycarbonylamino-, die tert.-Butyloxycarbonyloxy- und die Diphenylmethoxycarbonylgruppe gleichzeitig gespalten werden.

Salze von Verbindungen der Formel I und von Nitrilen der Formel $R^o$-CN können in an sich bekannter Weise hergestellt werden. So kann man Salze von solchen Verbindungen mit sauren Gruppen z.B. durch Behandeln mit Metallverbindungen, wie Alkalimetallsalzen von geeigne-

ten Carbonsäuren, z.B. dem Natriumsalz der α-Aethylcapronsäure, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen mit basischen Gruppierungen erhält man in üblicher Weise, z.B. durch Behandeln mit einer Säure oder einem geeigneten Anionenaustauschreagens. Innere Salze von Verbindungen welche z.B. eine salzbildende Aminogruppe und eine freie Carboxylgruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basische Mittel.

Erhaltene Gemische von Isomeren können nach an sich bekannten Methoden, in die einzelnen Isomeren getrennt werden, Gemische von diastereomeren Isomeren z.B. durch fraktioniertes Kristallisieren, Adsorptionschromatographie (Kolonnen- oder Dünnschichtchromatographie) oder andere geeignete Trennverfahren. Erhaltene Racemate können in üblicher Weise, gegebenenfalls nach Einführen von geeigneten salzbildenden Gruppierungen, z.B. durch Bilden eines Gemisches von diastereoisomeren Salzen mit optisch aktiven salzbildenden Mitteln, Trennen des Gemisches in die diastereomeren Salze und Umwandlung der Salze in die freien Verbindungen oder durch fraktioniertes Kristallisieren aus optisch aktiven Lösungsmitteln, in die Antipoden getrennt werden.

Das Verfahren umfasst auch diejenigen Ausführungsformen, wonach als Zwischenprodukte anfallende Verbindungen als Ausgangsstoffe verwendet und die restlichen Verfahrensschritte mit diesen durchgeführt werden, oder das Verfahren auf irgendeiner Stufe abgebrochen wird; ferner können Ausgangsstoffe in Form von Derivaten verwendet oder in situ, gegebenenfalls unter den Reaktionsbedingungen, gebildet werden.

Die Erfindung betrifft auch die neuen Verbindungen der Formel I, insbesondere diejenigen worin $R^1$ eine veresterte Hydroxycarbonylgruppe der Formel $R^1_a$-O-C(=O)- bedeutet.

Diese Verbindungen sind wertvolle Zwischenprodukte, die gegenüber den bekannten Verbindungen der Formel I, worin $R^1$ Wasserstoff ist, den Vorteil haben, dass sie stabiler sind und sie sich darum besser lagern lassen.

Bevorzugte neue Verbindungen der Formel I sind solche, worin $R^1$ eine veresterte Hydroxycarbonylgruppe der Formel $R^1_a$-O-C(=O)- ist, worin $R^1_a$ die genannten Bedeutungen hat, insbesondere Niederalkyl, bevorzugt tert.-Butyl, oder durch gegebenenfalls im Phenylring durch Halogen, Niederalkoxy oder Nitro substituiertes Benzyl ist, $R^2$ die genannten Bedeutungen hat, insbesondere eine α-Carboxy-4-hydroxybenzylgruppe ist, worin Carboxy und Hydroxy gegebenenfalls in geschützter Form vorliegen, Carboxy beispielsweise als Ester, wie Diphenylmethylester, und Hydroxy beispielsweise als tert.-Butyloxycarbonyloxy- oder Benzyloxycarbonyloxygruppe, und $R^3$ Wasserstoff bedeutet, und die am asymmetrischen C-Atom die Konformation der natürlich vorkommenden Nocardicine haben.

Die Erfindung betrifft ebenfalls die neuen Verbindungen der Formel $R^o$-CN, worin $R^o$ insbesondere ω-Carb-

oxy-ω-amino-niederalkoxyphenyl, worin Carboxy und Amino gegebenenfalls in geschützter Form vorliegen.

Diese Nitrile sind wertvolle Zwischenprodukte, die sich auf konventionellem Wege in die aus der Deutschen Offenlegungsschrift 2.714.628 bekannten 2-$R^o$-2-oxo- bzw. 2-$R^o$-2-hydroxyiminoessigsäuren überführen lassen, die ihrerseits wertvolle Acylierungsmittel bzw. Ausgangsmaterialien für die Synthese von 3-Acylamino-2-azetidinonverbindungen darstellen.

Die Ueberführung der Nitrile $R^o$-CN in die 2-Oxo- und in die 2-Hydroxyiminoessigsäuren kann beispielsweise nach dem folgenden Reaktionsschema erfolgen:

$R^o$-CN ⟶ $R^o$-CHO ⟶ $R^o$-CH(OH)-$CH_3$ ⟶
$R^o$-C(=O)-$CH_3$ ⟶ $R^o$-C(=O)-COOH ⟶ $R^o$-C(=N-OH)-COOH

Ein Aldehyd $R^o$-CHO wird erhalten, indem man ein Nitril $R^o$-CN, worin funktionelle Gruppen gegebenenfalls in geschützter Form vorliegen, mit einem Reduktionsmittel behandelt und das zunächst entstehende Zwischenprodukt hydrolysiert.

Geeignete Reduktionsmittel sind beispielsweise Lithium-triniederalkoxy-aluminiumhydrid, wie Lithium-triäthoxy-aluminiumhydrid, Zinn-II-chlorid, und katalytisch, z.B. mit $Pd(OH)_2/BaSO_4$ oder Raney-Nickel angeregter Wasserstoff. Die Reduktion wird in einem inerten Lösungsmittel, beispielsweise einem Aether oder im Falle der katalytischen Hydrierung auch in einem Alkohol, wie Methanol, gegebenenfalls in Gegenwart von Dianilinoäthan, bei Raumtemperatur oder leicht erniedrigter oder erhöhter Temperatur durchgeführt. Das zunächst entstehende Zwischenprodukt wird dann durch Wasser oder verdünnte Säuren zum Aldehyd hydrolysiert.

In einem erhaltenen Aldehyd $R^o$-CHO können die funktionellen Gruppen geschützt oder geschützte funktionelle Gruppen freigesetzt werden. Für die nachfolgenden

Reaktionen werden vorhandene Carboxylgruppen vorteilhafterweise verestert, beispielsweise mit einer Diazoverbindung, wie Diphenyldiazomethan.

Die neuen Aldehyde $R^o$-CHO und das Verfahren zu ihrer Herstellung sind ebenfalls Gegenstand der vorliegenden Erfindung.

Eine Hydroxyäthylverbindung $R^o$-CH(OH)-$CH_3$ wird erhalten, indem man einen Aldehyd $R^o$-CHO, worin funktionelle Gruppen gegebenenfalls in geschützter Form vorliegen, mit einer Methyl-Grignardverbindung behandelt.

Als Methyl-Grignardverbindungen dienen die üblichen Methylmagnesiumhalogenide, wie das Chlorid, Bromid oder Jodid. Im eingesetzten Aldehyd $R^o$-CHO liegen Carboxy- und Aminogruppen bevorzugt in geschützter Form vor.

Die Grignardreaktion wird auf übliche Weise in einem inerten Lösungsmittel, beispielsweise einem Aether, wie Diäthyläther und/oder Tetrahydrofuran, bei Raumtemperatur oder leicht erniedrigter oder erhöhter Temperatur durchgeführt.

Die neuen Hydroxyäthylverbindungen $R^o$-CH(OH)-$CH_3$ und das Verfahren zu ihrer Herstellung sind ebenfalls Gegenstand der Erfindung.

Ein Keton $R^o$-C(=O)-$CH_3$ wird erhalten, indem man eine Hydroxyäthylverbindung $R^o$-CH(OH)-$CH_3$ oxidiert.

Als Oxidationsmittel dienen die für die Ueberführung von sekundären Alkoholen in Ketone üblichen Reagentien, wie Alkalimetalldichromate, z.B. Natrium- oder Kaliumdichromat, Chromtrioxid, N-Halogenamide, wie N-Bromacetamid, N-Bromsuccinimid oder N-Chlorsuccinimid, Sulfoxide, wie Dimethylsulfoxid, und insbesondere Mangandioxid.

Die Oxidation wird in einem der üblichen Lösungsmittel bei Raumtemperatur oder leicht erniedrigter oder erhöhter Temperatur durchgeführt. Bevorzugt werden solche Oxidationsmittel und Lösungsmittel angewendet, bei denen

gegebenenfalls im Ausgangsmaterial vorhandene Schutzgruppen nicht abgespalten werden. Ein solches Oxidationssystem ist beispielsweise Mangandioxid in Methylenchlorid.

Falls gewünscht, können in einem erhaltenen Keton $R^o-C(=O)-CH_3$ vorhandene Schutzgruppen nach den üblichen Methoden abgespalten werden und/oder freie funktionelle Gruppen geschützt werden.

Das Verfahren zur Herstellung der Ketone $R^o-C(=O)-CH_3$ ist ebenfalls Gegenstand der vorliegenden Erfindung.

Gewisse Ketone $R^o-C(=O)-CH_3$ und alternative Verfahren zu ihrer Herstellung sind bereits aus der Deutschen Offenlegungsschrift 2.714.628 bekannt. Auch die Weiterverarbeitung zu den 2-Oxoessigsäuren $R^o-C(=O)-COOH$ und den 2-Hydroxyiminoessigsäuren ist aus der genannten Deutschen Offenlegungsschrift bekannt.

In den Verbindungen $R^o-CN$, $R^o-CHO$, $R^o-CH(OH)-CH_3$ ist $R^o$ bevorzugt ein in fermentativ erhältlichen Verbindungen der Formel II vorkommender Rest, insbesondere der 4-((3R)-3-Carboxy-3-aminopropoxy)phenylrest, worin Carboxy und Amino gegebenenfalls in geschützter Form vorliegen.

Die vorliegende Erfindung wird in den folgenden nicht-limitierenden Beispielen näher erläutert, worin folgende Abkürzungen verwendet wurden:

DC = Dünnschichtchromatogramm auf Silikagel.

Die Temperaturen sind in Celsiusgraden angegeben.

Beispiel 1: Eine Lösung von 1 g Natriumsalz von Nocardicin-A [M. Hashimoto, T. Komore und R. Kamiya, J. Amer. Chem. Soc. 98,3023 (1976)], in einer Suspension von 4 g Natriumcarbonat (38 mM) in 24 ml Wasser und 40 ml Dioxan wird bei Raumtemperatur während 10 Minuten mit 1,5 ml Di-tert.-butyldicarbonat (6,8 mM) versetzt. Das Gemisch wird auf 45° erwärmt und während 5 Tagen unter Rühren (ca. 1 Tropfen pro Stunde) weitere 4 ml Di-tert.Butyldicarbonat hinzugetropft. Nach dem Erkalten versetzt man das Gemisch mit weiteren 1 ml dieses Reagenzes und lässt noch 2 Stunden bei Raumtemperatur rühren.Man fügt 100 ml Wasser zum Gemisch und extrahiert zweimal mit je 200 ml Methylenchlorid. Die wässerige Phase kühlt man auf 0-5° und versetzt sie mit 170 ml eiskalter 5 proz.-Zitronensäure (pH des Gemisches 4). Man extrahiert dreimal mit je 500 ml Essigester, wäscht die organische Phase zweimal mit je 100 ml Sole, trocknet mittels Magnesiumsulfat und dampft ein wobei man 1,4 g eines Gemisches von 4-((3R)-3-tert.-Butyloxycarbonylamino-3-carboxypropoxy)benzonitril und (3S)-3- tert.-Butyloxycarbonylamino-(αR)-α-(4-hydroxyphenyl)-2-oxo-1-azetidinessigsäure erhält.Dieses Gemisch löst man in 10 ml Methanol und versetzt die Lösung mit 100 ml Aether und 1,5 g Diphenyldiazomethan (8,1 mM). Man lässt das Gemisch über Nacht bei Raumtemperatur rühren und dampft anderntags in vacuo ein, wobei man 2,8 g Rohgemisch erhält, welches man auf 100 g Kieselgel aufgezogen mit Toluol:Methylenchlorid 1:1 chromatographiert. Durch Eluieren mit Methylenchlorid erhält man 0,666 g (70,3 %) 4-((3R)-3-Diphenylmethoxycarbonyl-3-tert.-butyloxycarbonylaminopropoxy)benzonitril. IR-Absorptionsspektrum (in $CH_2Cl_2$): charakteristische Banden bei 3400, 2250, 1740, 1715 $cm^{-1}$; NMR-Spektrum ($DMSO-d_6$):

| δ-Werte (ppm) | Anzahl H | Zuordnung |
|---|---|---|
| 1,4 s | 9 | tert.-Butyl-$CH_3$ |
| 2,0-2,4 m | 2 | >CH-$CH_2$-$CH_2$ |
| 4,15 t | 2 | O-$CH_2$-$CH_2$; J = 6Hz |
| 4,2-4,5 m | 1 | -NH-CH-COOR |
| 7,15 und 7,75 q | 4 | 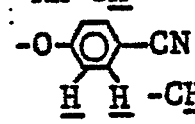 |
| 6,85 s | 1 | -CHØ$_2$ |
| 7,2-7,6 m | 10 | Phenyl-H + 1 NH |

Durch weiteres Eluieren mit Methylenchlorid erhält man 539 mg (46 %) (3S)-3-tert.-Butyloxycarbonylamino-(αR)-α-(4-tert.-butyloxycarbonyloxyphenyl)-2-oxo-1-azetidinessigsäure-diphenylmethylester. IR-Absorptionsspektrum (in $CH_2Cl_2$): charakteristische Banden bei 3400, 1760, 1720 $cm^{-1}$; NMR-Spektrum (DMSO-$d_6$):

| δ-Werte (ppm) | Anzahl H | Zuordnung |
|---|---|---|
| 1,38 s | 9 | tert.-Butyl-$CH_3$ |
| 1,49 s | 9 | tert.-Butyl-$CH_3$ |
| 3,1-3,2 m | 1 | 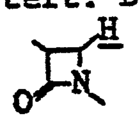 |
| 3,65-3,85 t | 1 | 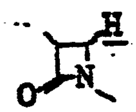 |
| 4,5-4,8 breit | 1 | H-3 |
| 5,82 s | 1 | 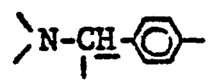 |
| 6,85 s | 1 | CHØ$_2$ |
| 7,0-7,8 m | 14 | Ar-H + 1 NH |

Durch weiteres Eluieren mit Methylenchlorid: Methanol 98:2 erhält man 0,422 g (46 % d.Th.) (3S)-3-tert.-Butyloxycarbonylamino-(αR)-α-(4-hydroxyphenyl)-2-oxo-1-azetidinessigsäure-diphenylmethylester. IR-Absorptionsspektrum (in $CH_2Cl_2$): charakteristische Banden

bei 3620, 3530, 1760, 1745, 1720 $cm^{-1}$; NMR-Spektrum ($DMSO-d_6$):

| δ-Werte (ppm) | Anzahl H | Zuordnung |
|---|---|---|
| 1,32 s | 9 | tert.-Butyl-$CH_3$ |
| 3,0 q | 1 | 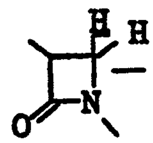 |
| 3,65 t | 1 | |
| 4,65 breit | 1 | H-3 |
| 5,60 s | 1 | N-CH-Ar |
| 6,84 s | 1 | $CH\Phi_2$ |
| 7,0-7,7 | 14 | ArH + 1 NH |

Beispiel 2: Eine Lösung von 160 g Natriumcarbonat in 600 ml Wasser wird mit 1200 ml Dioxan und 40 g Nocardicin-A (0,076 M) versetzt. Das Gemisch erwärmt man auf 30° und tropft während 30 Minuten 52 ml Di-tert.-Butyldicarbonat (0,238 M) hinzu. Nach dem Einstellen der Innentemperatur auf 55° tropft man weitere 52 ml (0,238 M) Di-tert.-Butyldicarbonat während 24 Stunden hinzu, wonach die Absorption bei 250 nm ein Maximum erreicht. Vor der Aufarbeitung kühlt man das Gemisch auf 30° und tropft während 24 Stunden weitere 40 ml (0,183 M) Di-tert.-Butyldicarbonat hinzu, wonach im DC nur noch zwei Flecken entsprechend den Produkten nachweisbar sind. Das Gemisch kühlt man auf 15-20° und nutscht die anorganischen Salze ab. Man wäscht den Rückstand gründlich mit Dioxan nach und dampft das Filtrat bei 10° (Trockeneiskühlfalle) auf ca. 750 ml ein. Man kühlt diesen Rückstand auf 0-5° und extrahiert dreimal mit je 500 ml Essigester. Die wässrige Phase stellt man bei 0-5° mittels konzentrierter Citronensäure auf pH 4 und extrahiert unverzüglich dreimal mit

je 600 ml Essigester. Die organische Phase wäscht man zweimal mit je 100 ml Sole, trocknet mit Magnesiumsulfat und dampft auf etwa 200 ml ein. Das ausgefallene Produkt wird abgenutscht und mit Essigester farblos gewaschen, wobei man 25,4 g (75% d.Th.) reine (3S)-3-tert.-Butyloxycarbonylamino-(αR)-α-(4-tert.-butyloxycarbonyloxyphenyl)-2-oxo-1-azetidinessigsäure erhält. Smp. 195° (mit Zersetzung); $[\alpha]_D^{20}$= -156 ± 1° (0,5 M $NaHCO_3$). IR-Absorptionsspektrum (Nujol): charakteristische Banden bei 3670, 1755, 1735, 1705 $cm^{-1}$; NMR-Spektrum (in DMSO-$d_6$):

| δ-Werte (ppm) | Anzahl H | Zuordnung |
|---|---|---|
| 1,37 s | 9 | t.-Butyl-$CH_3$ |
| 1,50 s | 9 | t.-Butyl-$CH_3$ |
| 3,10 q | 1 | 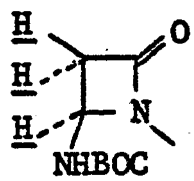 |
| 3,78 t | 1 | |
| 4,71 breit | 1 | |
| 5,50 s | 1 | N-CH-COOH |
| 7,23 und 7,45 q | 4 | Arom. $A^2B^2$ |
| 7,60 d | 1 | NH J = 8 Hz |

Aus der Mutterlauge kristallisiert nach Zugabe von wenig Aether 18,1 g (74% d.Th.) 4-((3R)-3-tert.-Butyloxycarbonylamino-3-carboxypropoxy)benzonitril vom Smp. 132-146°, welches man zur Analyse aus Ether umkristallisiert, wobei man ein Produkt vom Smp. 144-145° erhält. $[\alpha]_D^{20}$= - 8 ± 1° (0,5 M $NaHCO_3$). IR-Absorptionsspektrum (Nujol): charakteristische Banden bei 3400, 2270, 1775 sh, 1720, 1610 $cm^{-1}$, UV-Spektrum: $\lambda_{max}^{0,5\ M\ NaHCO_3}$ = 2500 nm (ε = 22 000)

| NMR-Spektrum (in DMSO-$d_6$) | δ-Werte (ppm) | Anzahl H | Zuordnung |
|---|---|---|---|
| | 1,38 s | 10 | t.-Butyl-$CH_3$ |
| | 1,95-2,35 breit | 2 | $CH-CH_2-CH_2$ |
| | 4,14 t | 3 | $CH-CH_2$+ $O-CH_2-CH_2$ J = 6 Hz) |
| | 7,08 und 7,71 q | 4 | Arom. $A^2B^2$ (J = 9 Hz) |

Beispiel 3: 20 g Nocardicin-A (0,038 Mol) werden in einer Suspension von 80 g $Na_2CO_3$ (0,76 Mol) in 300 ml Wasser und 600 ml Dioxan gelöst und bei 30° während 10 Minuten mit 26 ml (0,117 Mol) Di-tert.butyldicarbonat versetzt. Das Gemisch wird auf 40° erwärmt und unter Rühren während 9 Tagen (ca. 4 Tropfen pro Stunde) mit weiteren 54 ml (0,243 Mol) Di-tert.butyldicarbonat versetzt. Bei Badtemperatur 25° werden 400 ml Wasser-Dioxan Gemisch am Wasserstrahlvacuum abdestilliert. Durch Zugabe von 4 Liter Wasser bringt man den Rückstand in Lösung und extrahierte diese viermal mit je 500 ml Methylenchlorid. Die organische Phase wird mit 1 Liter Wasser gewaschen, wobei eine Emulsion ensteht, welche sich aber nach wenigen Minuten trennt. Die wässrige Phase versetzt man mit Eis und überschichtet sie mit 4 Liter eisgekühltem Essigsäureäthylester. Bei 0-5° stellt man das Gemisch mit 10 proz. Citronensäure auf pH 4. Die wässrige Phase wird noch zweimal mit je 3 Liter Essigsäureäthylester nachgewaschen. Die organische Phase wird mit je zweimal 1 Liter Sole gewaschen, über $MgSO_4$ getrocknet und bei 25° in vacuo eingedampft, wobei man ein Gemisch bestehend aus dem 4-((3R)-3-tert.-Butyloxycarbonylamino-3-carboxypropoxy)benzonitril, (3S)-3-tert.-Butyloxycarbonylamino-(αR)-α-(4-tert.-butyloxycarbonyloxyphenyl)-2-oxo-1-azetidinessigsäure und (3S)-3-tert.-Butyloxycarbonylamino-(αR)-α-(4-hydroxyphenyl)-2-oxo-1-azetidinessigsäure erhält.

26,3 g dieses Säuregemisches werden in 1 Liter Dioxan gelöst und mit 20 g Diphenyldiazomethan versetzt (0,1 Mol). Die dunkelrote Lösung rührte man über Nacht bei Zimmertemperatur, wobei nach DC sämtliches Ausgangsmaterial umgesetzt wird. Das leicht rot gefärbte Gemisch dampft man in vacuo ein und erhält 47 g rohes Estergemisch, welches man auf 2 kg Kieselgel reinst (Merck) aufgezogen mit Toluol:Methylenchlorid 1:1 chromatographiert. Durch Eluieren der Säule mit Methylenchlorid erhält man 11,9 g (65% roh) 4-((3R)-3-Diphenylmethoxycarbonyl-3-tert.-butyloxycarbonylaminopropoxy)benzonitril, aus dem man durch Kristallisation aus Aether 9,5 g (52% d.Th.) Reinprodukt vom Smp. 127-129°, $[\alpha]_D^{20}$=-3±1° (1% in Chloroform) erhält. Die IR-, UV- und NMR-Spektren sind mit denjenigen des gemäss Beispiel 1 erhaltenen Produktes identisch.

Durch weiteres Eluieren erhält man 9,5 g (42% roh) (3S)-3-tert.-Butyloxycarbonylamino-(αR)-α-(4-tert.-butyloxycarbonyloxyphenyl)-2-oxo-1-azetidinessigsäure-diphenylmethylester aus dem man durch Kristallisation aus Aether, gefolgt durch Kristallisation aus abs. Aethanol 3,8 g (17% d.Th.) Reinprodukt vom Smp. 155-157° erhält, welches identisch ist mit dem Produkt aus dem Beispiel 1.

Durch weiteres Eluieren mit Methylenchlorid: Methanol 98:2 erhält man 7,7 g (40% roh) (3S)-3-tert.-Butyloxycarbonylamino-(αR)-α-(4-hydroxyphenyl)-2-oxo-1-azetidinessigsäure-diphenylmethylester, aus dem man durch Kristallisation aus Aether 5,3 g (28% d.Th.) Reinprodukt vom Smp. 174-176°, $[\alpha]_D^{20}$ = -115±1° (1% in Chloroform) erhält. Das IR- und NMR-Spektrum sind mit dem gemäss Beispiel 1 erhaltenen Produkt identisch.

Beispiel 4: 20 g Nocardicin-A (38 mM) werden in einer Suspension von 80 g $Na_2CO_3$ in 600 ml Dioxan und 300 ml Wasser gelöst. Das Gemisch erwärmt man auf 55° und tropft während 6 Stunden unter Rühren 26 ml (0,117 Mol) Di-tert.-butyldicarbonat dazu. Während weiteren 20 Stunden tropft man abermals 26 ml (0,11 Mol) Di-tert.-butyldicarbonat hinzu, wonach der "Absorbancewert" einer 1 ml Probe verdünnt auf 1 Liter 0,63 (entsprechend 70% Umsatz) beträgt. Das Gemisch kühlt man auf 30°,gibt innert 10 Minuten weitere 10 ml (0,045 Mol) Di-tert.-butyldicarbonat zu und erwärmt wieder während 16 Stunden auf 55°(gemäss UV-Spektrum 79% Umsatz). Dieser Vorgang wird noch zweimal wiederholt, wonach der "Absorbancewert" 0,8 (entsprechend 90% Umsatz) beträgt. Zur Vervollständigung der Veresterung gibt man zum Gemisch bei Raumtemperatur aufs Mal 40 g $Na_2CO_3$ und 8 ml (0,036 Mol) Di-tert.-butyldicarbonat. Zwei weitere Portionen von je 8 ml werden in Abständen von jeweils 24 Stunden aufs Mal zugegeben, wonach DC (Chloroform:Methanol:Eisessig 25:12:3) den vollständigen Umsatz von freiem Phenol anzeigt (Totalverbrauch an Di-tert.-butyldicarbonat = 0,477 Mol).

Das Gemisch wird auf 0-5° gekühlt, abgenutscht und der Rückstand mit 400 ml Dioxan:Wasser 2:1 gründlich gewaschen. Bei einer Badtemperatur unter 10° (Trockeneis Kühlfalle) wird das Gemisch in vacuo auf die Hälfte des Volumens eingedampft und auf 0-5° gekühlt. Nach zweimaligem Extrahieren mit je 500 ml eisgekühltem Essigester stellt man die wässrige Lösung mit 10 prozentiger Citronensäure auf pH 4. Das Gemisch wird unverzüglich mit je drei 1500 ml Portionen vorgekühltem Essigester extrahiert. Die organische Phase wird mit je zwei 250 ml Portionen vorgekühlter Sole gewaschen, über Magnesiumsulfat kurz getrocknet und in vacuo bis auf ca. 200 ml eingedampft. Das ausgefallene Produkt wird abgenutscht, mit Essigester und absolutem Aether gewaschen und über Nacht am Hochvakuum getrocknet,wobei man 8,9 g,(53% d.Th.) reine (3S)-3-tert.-Butyloxycarbonylamino-(αR)-α-(4-tert.-butyloxycarbonyloxyphenyl)-2-oxo-1-azetidinessigsäure vom Smp. 195° (mit Zersetzung), $[\alpha]_D^{20}$=-156±1° (in 0,5 M $NaHCO_3$) erhält.

Beispiel 5: 2 g (4,49 mM) (3S)-3-tert.-Butyloxycarbonylamino-(αR)-α-(4-tert.-butyloxycarbonyloxyphenyl)-2-oxo-1-azetidinessigsäure werden in 50 ml Dioxan gelöst und diese Lösung aufs Mal mit 1 g (5,2 mM) Diphenyldiazomethan versetzt. Nach 4 Stunden zeigt sich im DC ($CHCl_3$:MeOH:HOAc 85:12:3) kein Ausgangsmaterial mehr. Die rote Farbe wird entfernt durch Zugabe von zwei Tropfen Eisessig und das Gemisch bei Badtemperatur 25° im Vakuum eingedampft. Den farblosen Schaum löst man in 20 ml absolutem Aethanol, wobei das Produkt auszukristallisieren beginnt. Man erhält 2,4 g (87% d.Th.) reinen (3S)-3-tert.-Butyloxycarbonylamino - (αR)-α-(4-tert.-butyloxycarbonyloxyphenyl)-2-oxo-1-azetidinessigsäure-diphenylmethylester vom Smp. 155-157°.

Beispiel 6: Eine Lösung von 12 g Natriumcarbonat in 30 ml Wasser wird mit 30 ml Dioxan und 2,0 g (0,0038 Mol) Nocardicin-A versetzt. Bei 0-5° werden während 15 Minuten 5 ml einer Lösung von 3,6 g (0,021 Mol) Carbobenzoxychlorid in 25 ml Dioxan zugetropft. Den Rest der Lösung tropft man während 24 Stunden bei 60° hinzu und rührt anschliessend während drei Tagen bei dieser Temperatur weiter. Das Gemisch kühlt man auf 35° und tropft während 24 Stunden 4,8 ml Di-tert.-butyl-dicarbonat hinzu. Man nutscht ab, wäscht mit Dioxan:Wasser 1:1 und dampft bei

einer Temperatur unter 20° das Filtrat ein. Den Rückstand schlämmt man in 100 ml Eiswasser auf und extrahiert dreimal mit Essigester. Bei 0-5° wird die wässrige Phase mit 2N Citronensäure auf pH 4 gestellt und mit vorgekühltem Essigester dreimal extrahiert. Die organische Phase wäscht man zweimal mit kalter Natriumchloridlösung, trocknet über Natriumsulfat und dampft ein wobei man 2,2 g eines Gemisches von (3S)-3-Benzyloxycarbonylamino-(αR)-α-(4-tert.-butyloxycarbonyloxyphenyl)-2-oxo-1-azetidinessigsäure und 4-((3R)-3-Benzyloxycarbonylamino-3-carboxypropoxy)benzonitril in Form eines starren Schaums erhält.

Das erhaltene Rohgemisch wird in 70 ml Dioxan gelöst, mit 1 g Diphenyldiazomethan (0,00514 Mol) versetzt.und während drei Tagen bei Raumtemperatur gerührt. Die Reaktionsmischung wird durch Zugabe von wenig Eisessig entfärbt, eingedampft, auf 100 g Kieselgel aufgezogen und mit Toluol chromatographiert. Durch Eluieren mit Toluol:Essigester 98:2 erhält man 1,8 g 4-((3R)-3-Benzyloxycarbonylamino-3-diphenylmethoxycarbonylpropoxy)benzonitril, welches man aus Aether umkristallisiert und welches mit dem im Beispiel 7 beschriebenen Produkt identisch ist. Durch weiteres Eluieren mit Toluol:Essigester 96:4 erhält man 1,0 g (3S)-3-Benzyloxycarbonylamino-(αR)-α-(4-tert.-butyloxycarbonyloxyphenyl)-2-oxo-1-azetidinessigsäure-diphenylmethylester welches man aus Aether umkristallisiert. Smp. 153-154°; $[\alpha]_D^{20} = -99 \pm 1°$ IR-Absorptionsspektrum (in $CH_2Cl_2$): charakteristische Banden bei 3430, 1767, 1750 sh, 1732 sh, 1510 $cm^{-1}$;

| NMR-Spektrum (DMSO-$d_6$) | δ-Werte (ppm) | Anzahl H | Zuordnung |
|---|---|---|---|
| | 1,50 s | 9 | $-C(C\underline{H}_3)_3$ |
| | 3,0-3,2 m | 1 | 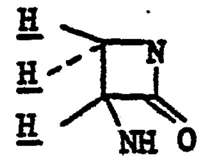 |
| | 3 t (J=6+6Hz) | 1 | |
| | 4,6-4,9 m | 1 | |
| | 5,02 s | 2 | $NHCOOC\underline{H}_2\phi$ |
| | 5,86 s | 1 | $-C\underline{H}COOR$ |
| | 6,88 s | 1 | $C\underline{H}\phi_2$ |
| | 7,0-7,5 m | 19 | Ar-$\underline{H}$ |
| | 8,02 d | 1 | $N\underline{H}$ |

Beispiel 7: Eine Lösung von 24 g Natriumcarbonat in 60 ml Wasser wird mit 60 ml Dioxan und 4 g (0,0076 Mol) Nocardicin-A versetzt. Bei 0-5° werden während 15 Minuten 10 ml einer Lösung von 12 g Carbobenzoxychlorid (0,07 Mol) in 90 ml Dioxan zugetropft. Das Gemisch wird anschliessend auf 60° erwärmt und weitere 50 ml der Carbobenzoxychloridlösung während 24 Stunden zugetropft. Das Gemisch wird anschliessend wieder auf Raumtemperatur gekühlt und während 3 Stunden mit dem Rest der Carbobenzoxychloridlösung behandelt. Man lässt das Gemisch über Nacht weiterrühren und dampft bei Raumtemperatur (20°) ein. Den Rückstand löst man in Wasser und extrahiert dreimal mit je 400 ml Essigester ohne die organische Phase zu waschen. Die Essigesterphase, welche die löslichen Natriumsalze der Produkte enthält, wird eingedampft und der semikristalline Rückstand (14 g) in 200 ml Wasser aufgeschlämmt. Mit 2N Salzsäure wird bei 0-5° der pH auf 3,5 eingestellt und dreimal mit je 200 ml Essigester extrahiert. Die organische Phase trocknet man über Magnesiumsulfat und dampft ein, wobei man 6 g eines Rohgemisches

von (3S)-3-Benzyloxycarbonylamino-(αR)-α-(4-benzyloxycarbonyloxyphenyl)-2-oxo-1-azetidinessigsäure und 4-((3R)-3-Benzyloxycarbonylamino-3-carboxypropoxy)benzonitril erhält.

Das erhaltene Rohgemisch wird in 100 ml Dioxan gelöst und mit 3 g (0,0154 Mol) Diphenyldiazomethan versetzt. Das Gemisch wird während zwei Tagen bei Raumtemperatur gerührt, mit fünf Tropfen Eisessig versetzt und noch zwei Stunden weitergerührt. Nach dem Eindampfen erhält man 8 g eines roten Harzes, welches man auf 400 g Kieselgel chromatographiert. Durch Eluieren mit Toluol:Essigester 98:2 erhält man 0,3 g 4-((3R)-3-Benzyloxycarbonylamino-3-diphenylmethoxycarbonylpropoxy)benzonitril, welches man aus Aether umkristallisiert; Smp. 125-126°; $[\alpha]_D^{20} = +1\pm1°$; IR-Absorptionsspektrum (in $CH_2Cl_2$): charakteristische Banden bei 3460, 2240, 1748s, 1737, 1610, 1510 $cm^{-1}$;

| NMR-Spektrum ($CDCl_3$) | δ-Werte (ppm) | Anzahl H | Zuordnung |
|---|---|---|---|
| | 2,0-2,4 m | 2 | CH-C$\underline{H}_2$-$CH_2$ |
| | 4,10 t (J = 6 Hz) | 2 | -O-C$\underline{H}_2$-$CH_2$ |
| | 4,2-4,6 m | 1 | -C$\underline{H}$-$CH_2$ |
| | 5,01 s | 2 | COOC$\underline{H}_2$Ø |
| | 6,8 s | 1 | CHØ$_2$ |
| | 6,98 und 7,69 q | 4 | Arom. $A^2B^2$ |
| | 7,29 s | 15 | restl. Arom. |
| | 7,92 d (J = 8 Hz) | 1 | N$\underline{H}$ |

Durch weiteres Eluieren mit Toluol:Essigester 95:5 erhält man 1,7 g (3S)-3-Benzyloxycarbonylamino-(αR)-

α-(4-benzyloxycarbonyloxyphenyl)-2-oxo-1-azetidinessigsäure-diphenylmethylester, welchen man aus Aether umkristallisiert. Smp. 95-100° (unter Zersetzung); $[\alpha]_D^{20}$ = -90±1°; IR-Absorptionsspektrum (in $CH_2Cl_2$): charakteristische Banden bei 3450, 1770, 1752 sh, 1735 sh, 1510 $cm^{-1}$.

| NMR-Spektrum ($CDCl_3$) | δ-Werte (ppm) | Anzahl H | Zuordnung |
|---|---|---|---|
| | 3,10 q (J=3+6 Hz) | 1 | 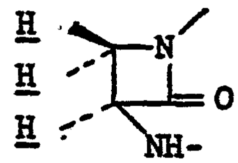 |
| | 3,87 t (J=6+6 Hz) | 1 | |
| | 4,65-4,93 m | 1 | |
| | 5,09 s | 2 | O-$COOCH_2$Ø |
| | 5,31 s | 2 | $NHCOOCH_2$Ø |
| | 5,45 d (J=8Hz) | 1 | -NH- |
| | 6,96 s | 1 | -CHØ$_2$ |
| | 7,0-7,5 m | 24 | arom. H |

Beispiel 8: Eine Suspension von 160 g (0,31 Mol) Nocardiacin-A-monohydrat (freie Säure) in 1,6 Liter Wasser wird durch Zugabe von gesättigter Natriumhydroxydlösung auf pH 10,2 gestellt. Zur klaren farblosen Lösung gibt man 1 Liter Dioxan und 200 ml (0,99 Mol) Di-tert.Butyldicarbonat und rührt während 3 Stunden bei 55°. Den pH-Wert hält man während der ganzen Zeit der Reaktion durch Zugabe von konzentrierter Natronlauge mittels Titrators zwischen 9,8 und 10,2. Anschliessend tropft man während weiteren 4 Stunden zusätzlich 150 ml (0,74 Mol) Di-tert.Butyldicarbonat hinzu, wonach die Absorption bei 250 nm ein Maximum erreicht. Vor der Aufarbeitung kühlt man das Gemisch auf 30° und tropft während 24 Stunden weitere 250 ml (1,24 Mol) Di-tert.Butyldicarbonat hinzu. Man nutscht ab, wäscht den Rückstand mit 500 ml Dioxan nach

und dampft das Filtrat im Vakuum bei 20° auf 2,5-3 Liter ein. Das Gemisch extrahiert man mit 1 Liter Essigester, kühlt auf 5° und säuert mittels konzentrierter Salzsäure auf pH 4,2 an. Nach wenigen Minuten kristallisiert das Produkt aus. Man nutscht ab und wäscht den Rückstand mit 500 ml Wasser und 1500 ml Essigester portionenweise nach, wobei man 108,1 g (80% d.Th.) (3S)-3-tert.-Butyloxycarbonylamino-(αR)-α-(4-tert.-butyloxycarbonyloxyphenyl)-2-oxo-1-azetidinessigsäure vom Smp. 173° (Zersetzung) erhält.

Aus den Essigester Waschlösungen erhält man nach dem Eindampfen auf ca. 200 ml 48 g (51% d.Th.) 4-((3R)-3-tert.-Butyloxycarbonylamino-3-carboxypropoxy) benzonitril vom Smp. 132-146°.

Beispiel 9: Eine Mischung von 1,5 g (3S)-3-(tert.-Butyloxycarbonylamino)-(αR)-α-(4-tert.-butyloxycarbonyloxyphenyl)-2-oxo-1-azetidinessigsäure-diphenylmethylester in 45 ml Acetonitril und 1,5 g p-Toluolsulfonsäure-monohydrat wird 75 Minuten bei Zimmertemperatur gerührt, wobei das Toluolsulfonsäuresalz vom (3S)-3-Amino-(αR)-α-(4-tert.-butyloxycarbonyloxyphenyl)-2-oxo-1-azetidinessigsäure-diphenylmethylester zum Teil auskristallisiert. Man verdünnt mit Essigester, stellt mit 1 N wässriger $NaHCO_3$-Lösung basisch, wäscht mit gesättigter wässriger NaCl-Lösung neutral, trocknet über $Na_2SO_4$ und dampft im Vakuum ein. Das anfallende Rohprodukt wird an 100 g Kieselgel chromatographiert. Dabei eluiert man mit Toluol-Essigester-(4:1)-Gemisch 111 mg des nicht umgesetzten Ausgangsesters. Spätere Toluol-Essigester-(1:1)- und Essigester-Fraktionen liefern 950 mg (3S)-3-Amino-(αR)-α-(4-tert.-butyloxycarbonyloxyphenyl)-2-oxo-1-azetidinessigsäure-diphenylmethylester; Smp. 120-122° nach Kristallisation aus Aether. IR-Spektrum ($CH_2Cl_2$): charakteristi-

Banden bei 3400-2500 (breit), 1760 (Schulter), 1750 $cm^{-1}$; UV-Spektrum (in Aethanol): $\lambda_{max}$ bei 252 nm ($\mathcal{E}$=620), 257 nm ($\mathcal{E}$=720) sowie Schultern bei 263 und 267 nm, sowie eine starke Endabsorption; NMR-Spektrum (in $CDCl_3$; ppm): 1,57, s, $C(CH_3)_3$; 3,05, dd, $J_{2,2}$ = 5, $J_{2,3}$ = 2,5, CH-2; 3,81, t, $J_{2,2}$ = $J_{2,3}$ = 5, CH-2; 4,32, bm, CH-3; 5,64, s, CHCOO; 6,87, s, $CHPh_2$; 7,00-7,30, div. m, 14-arom.H.

Beispiel 10: 500 mg (3S)-3-tert.-Butyloxycarbonylamino-(αR)-α-(4-tert.-butyloxycarbonyloxyphenyl)-2-oxo-1-azetidinessigsäure werden in 10 ml Trifluoressigsäure 10 Minuten bei 0° gerührt. Darauf verdünnt man mit Essigester und wäscht dreimal mit Wasser. Die vereinigten Wasserauszüge werden anschliessend durch Zugabe von Amberlite IR 45 (OH-Form) auf pH 4,5 gestellt. Man filtriert vom Ionenaustauscher ab, wäscht mit Wasser nach und dampft das erhaltene Filtrat im Vakuum bei einer Badtemperatur von 50° weitgehend ein. Durch Zugabe von Aethanol zu der auf ca. 5 ml eingeengten Wasserlösung erfolgt Kristallisation der (3S)-3-Amino-(αR)-α-(4-hydroxyphenyl)-2-oxo-1-azetidinessigsäure, die anschliessend abfiltriert, mit Aether gewaschen und am Hochvakuum getrocknet wird. Ausbeute 80 mg. Smp.: Zersetzung ab 200°. IR-Spektrum (in Nujol): charakteristische Baden bei 3175, 2688, 2597, 2500, 2325, 1776, 1754, 1639, 1589, 1519, 1499, 1464 $cm^{-1}$; NMR-Spektrum ($D_2O$ + 1 Aequivalent $NaHCO_3$): 3,30, dd, $J_{2,2}$ = 5, $J_{2,3}$ = 2,5 CH-2; 4,10, t, $J_{2,2}$ = $J_{2,3}$ = 5 CH-2; 4,64, dd, $J_{2,3}$ = 5 bzw. 2,5, CH-3; 5,57, s, CHCOO; 7,21 + 7,53, 2d,J = 8,4-arom. H.

Beispiel 11: Eine Mischung von 700 mg (3S)-3-(tert.-Butyloxycarbonylamino)-(αR)-α-(4-tert.-butyloxycarbonylphenyl)-2-oxo-1-azetidinessigsäure-diphenylmethylester und 1,5 ml Anisol in 20 ml $CF_3COOH$ wird 10 Minuten bei 0° gerührt. Man dampft im Vakuum (Badtemperatur 30°) zur Trockne ein, nimmt hintereinander je dreimal in Chloroform und Toluol auf und dampft jeweils im Vakuum zur Trockne ein. Anschliessend wird 1 Stunde am Vakuum getrocknet, der Rückstand in Aether digeriert, vom Aether abfiltriert und wiederum 1 Stunde am Vakuum getrocknet. Darauf löst man in 5 ml Methanol, fügt 50 ml Wasser zu und extrahiert mit Essigester, wobei zusätzlich dreimal mit Wasser gewaschen wird. Die vereinigten Wasseranteile werden durch Zugabe von Amberlite IR 45 (OH-Form) auf pH 4,5 gestellt und anschliessend durch Filtration vom Ionenaustauscher befreit, wobei gründlich mit Wasser nachgewaschen wird. Dann engt man im Vakuum auf 5 ml ein (Badtemperatur 50°) und kristallisiert durch Zugabe von Aethanol. Die kristalline (3S)-3-Amino-(αR)-α-(4-hydroxyphenyl)-2-oxo-1-azetidinessigsäure wird abfiltriert, mit Aether gewaschen und am Hochvakuum getrocknet. Ausbeute: 137 mg. Smp: Zersetzung ab 200°.

Beispiel 12: 700 mg (3S)-3-Amino-(αR)-α-(4-tert.-butyloxycarbonyloxyphenyl)-2-oxo-1-azetidinessigsäure-diphenylmethylester werden wie im Beispiel 11 umgesetzt und aufgearbeitet. Dabei resultieren 93 mg (3S)-3-Amino-(αR)-α-(4-hydroxyphenyl)-2-oxo-1-azetidinessigsäure. Smp: ab 200° Zersetzung.

Beispiel 13: 700 mg (3S)-3-tert.-Butyloxycarbonylamino-(αR)-α-(4-hydroxyphenyl)-2-oxo-1-azetidinessigsäure-diphenylmethylester werden wie im Beispiel 11 umgesetzt und aufgearbeitet. Dabei resultieren 40 mg (3S)-3-Amino-(αR)-α-(4-hydroxyphenyl)-2-oxo-1-azetidinessigsäure, Smp.: ab 200° Zersetzung.

Beispiel 14: Eine Lösung von 5,95 g (0,0098 Mol) (3S)-3-(tert.-Butyloxycarbonylamino)-(αR)-α-(4-tert.-butyloxycarbonyloxyphenyl)-2-oxo-1-azetidinessigsäure-diphenylmethylester in 180 ml absoluten Acetonitril wird bei Zimmertemperatur unter Rühren mit 5,95 g (0,03 Mol) p-Toluolsulfonsäure-monohydrat versetzt.

Die so erhaltene gelbe Lösung wird 75 Minuten bei Zimmertemperatur weitergerührt, wobei bereits nach 20 Minuten ein Teil des p-Toluolsulfonats auskristallisiert. Anschliessend wird auf -10° abgekühlt, mit 400 ml absolutem Aether versetzt und die Kristallisation durch 1-stündiges Rühren bei -10° vervollständigt. Das so erhaltene Produkt wird abgenutscht, mit absolutem Aether gewaschen und im Hochvakuum bei 50° getrocknet. Nach einmaliger Umkristallisation aus Isopropanol erhält man 2,9 g (43,5% d.Th.) reines p-Toluolsulfonat des (3S)-3-Amino-(αR)-α-(4-tert.-butyloxycarbonyloxyphenyl)-2-oxo-1-azetidinessigsäure-diphenylmethylesters in Form weisser Kristalle vom Smp. 150-151° (Zersetzung).

Beispiel 15: In 100 ml Trifluoressigsäure werden bei 0-5° 20 g (3S)-3-tert.-Butyloxycarbonylamino-(αR)-α-(4-tert.-butyloxycarbonyloxyphenyl)-2-oxo-1-azetidinessigsäure eingetragen. Nach Beendigung der $CO_2$-Entwicklung (~8 Minuten) wird die Reaktionsmischung am Hochvakuum

von überschüssiger Trifluoressigsäure weitgehend befreit. Der Rückstand wird mit 100 ml trockenem Aether digeriert, abfiltriert, mit Aether gewaschen und im Vakuum bei Raumtemperatur getrocknet. Das erhaltene Trifluoressigsäuresalz der (3S)-3-Amino-(αR)-α-(4-hydroxyphenyl)-2-oxo-1-azetidinessigsäure wird in 80 ml Methanol gelöst und die Lösung durch Zugabe von 30%-iger methanolischer Triäthylaminlösung auf pH 4,3 gestellt. Die auskristallisierende (3S)-3-Amino-(αR)-α-(4-hydroxyphenyl)-2-oxo-1-azetidinessigsäure wird abfiltriert und mit Methanol gewaschen; Smp.: 196-200°; Ausbeute 9,3 g (~ 86% d.Th.).

<u>Beispiel 16:</u> Eine Suspension von 9 g (21 mM) (3S)-3-tert.-Butyloxycarbonylamino-(αR)-α-(4-tert.-butyloxycarbonyloxyphenyl)-2-oxo-1-azetidinessigsäure und 2,23 g (21 mM) $Na_2CO_3$ in 60 ml $H_2O$ wird 2 Stunden bei 60° erwärmt (pH 9). Nach 2 Stunden wird filtriert und die klare farblose Lösung mit 2n HCl auf pH 2 gestellt. Der Niederschlag wird abfiltriert,mit Wasser und Essigester gewaschen und aus Essigester und Methanol umkristallisiert. Man erhält 5,8 g (~ 82%) (3S)-3-(tert.-Butyloxycarbonylamino)-(αR)-α-(4-hydroxyphenyl)-2-oxo-1-azetidinessigsäure. Smp.: ab 201° Zersetzung.

<u>Beispiel 17:</u> 4,55 g (3S)-3-(tert.-Butyloxycarbonylamino)-(αR)-α-(4-hydroxyphenyl)-2-oxo-1-azetidinessigsäure werden in 100 ml Methanol gelöst und bei Zimmertemperatur mit 5 g Diphenyldiazomethan in 30 ml Chloroform versetzt. Man rührt 3 Stunden bei Zimmertemperatur, zersetzt den Ueberschuss an Diphenyldiazomethan mit Eisessig, nimmt in Essigester auf und wäscht nacheinander mit gesättigten wässerigen Lösungen von $NaHCO_3$ und NaCl (bis zum Neutralpunkt). Darauf trocknet man über $Na_2SO_4$, dampft

im Vakuum ein und chromatographiert das anfallende Rohprodukt an 250 g Kieselgel. Die einheitlichen Toluol-Essigester-(1:1) Eluate werden vereinigt, eingedampft und anschliessend aus Aceton-Petroläther umkristallisiert. Man erhält 6,4 g (3S)-3-tert.-Butyloxycarbonylamino-(αR)-α-(4-hydroxyphenyl)-2-oxo-1-azetidinessigsäure-diphenylmethylester vom Smp. 176-178° (Zersetzung) $[\alpha]_D^{20} = -111°$ (c = 0,92 in $CHCl_3$). IR-Spektrum ($CH_2Cl_2$): charakteristische Banden bei 3580, 3420, 1760, 1742, 1720, 1618, 1600, 1518 $cm^{-1}$.

Beispiel 18: Eine Lösung von 1,8 g (5,5 mM) 4-((3R)-3-tert.Butoxycarbonylamino-3-carboxypropoxy)-benzonitril in 20 ml Methanol wird mit 1,0 ml Aethyldiisopropylamin versetzt und 15 Minuten bei Zimmertemperatur gerührt. Nach Zugabe von 1,2 g Dianilinoäthan-dihydrochlorid und 1,5 g Raney Nickel hydriert man bis zum Stillstand. Man filtriert den Katalysator ab, dampft ein und verteilt den harzigen Rückstand zwischen je 50 ml eisgekühlte 2-n. Salzsäure und Chloroform. Die organische Phase wäscht man mit eisgekühlter 2-n. Salzsäure und Wasser nach, trocknet sie über Magensiumsulfat und dampft ein, wobei man 0,9 g festen Rückstand erhält. Man kristallisiert aus Isopropanol-Aether um und erhält den 4-((3R)-3-tert.Butoxycarbonylamino-3-carboxypropoxy)-benzaldehyd vom Smp. 147-149° (mit Zersetzung); $[\alpha]_D^{20} = -7 \pm 1°$, (0,523% in 0,5 n-$NaHCO_3$); IR-Spektrum (in Dioxan): charakteristische Absorptionsbanden bei 3210, 1740, 1715, 1700, 1605, 1580 $cm^{-1}$.

Beispiel 19: Eine Lösung von 2,0 g (6,19 mM) 4-((3R)-3-tert.-Butoxycarbonyl-amino-3-carboxypropoxy)-benzaldehyd in 50 ml Dioxan wird mit 1,2 g (6,67 mM) Diphenyldiazomethan versetzt und 24 Stunden bei Raumtemperatur gerührt. Man dampft ein und verrührt den harzigen Rückstand wiederholt mit wenig Cyclohexan, wobei der Rückstand beginnt fest zu werden. Man erhält durch Abnutschen ein Rohprodukt, welches man aus Aether umkristallisiert, wobei man den 4-((3R)-3-tert.-Butoxycarbonylamino-3-benzhydryloxycarbonylpropoxy)-benzaldehyd vom Smp. 116-119° erhält; $[\alpha]_D^{20} = -5 \pm 1°$ (0,588 % in $CHCl_3$); IR-Spektrum (in $CH_2Cl_2$): charakteristische Absorptionsbanden bei 3420, 2730, 1740, 1712, 1695, 1605, 1580 $cm^{-1}$.

Beispiel 20: In einer trockenen Apparatur werden 0,476 g (0,97 mM) 4-((3R)-3-tert.Butoxycarbonylamino-3-benzhydryloxycarbonylpropoxy)-benzaldehyd in einem Gemisch von 25 ml Aether und 3 ml Tetrahydrofuran gelöst. Unter Stickstoff als Schutzgas wird bei 0-5° während 15 Minuten 3,3 mM Methylmagnesiumjodid, frisch hergestellt aus 0,081 g Magnesiumspänen und 0,2 ml Methyljodid in 10 ml Aether, hinzugetropft und anschliessend die Temperatur auf Raumtemperatur erhöht. Man giesst das Gemisch auf 50 ml 10 %-ige Ammoniumchloridlösung, welche wenig Eis enthält und trennt die wässrige Phase ab. Die organische Phase wäscht man mit Wasser nach und trocknet sie über Magnesiumsulfat. Nach dem Eindampfen erhält man ein farbloses Gemisch der zwei diastereomeren 4-((3R)-3-tert.Butoxycarbonylamino-3-benzhydryloxycarbonylpropoxy)-phenylmethylcarbinole welche ohne weitere Reinigung mit Mangandioxid oxidiert werden können.

Beispiel 21: 0,70 g (1,38 mM) 4-((3R)-3-tert.Butoxycarbonylamino-3-benzhydrylcarbonylpropoxy)-phenylmethylcarbinol (Diastereomerengemisch) werden in 100 ml Methylenchlorid gelöst, die Lösung mit 3 g Mangandioxid versetzt

und das Gemisch über Nacht bei Raumtemperatur gerührt. Man filtriert ab und dampft das Filtrat ein, wobei man einen gelben erstarrten Schaum erhält. Durch Zugabe von wenig Aether erhält man das 4-((3R)-3-tert.Butoxycarbonylamino-3-benzhydryloxycarbonylpropoxy)-phenylmethylketon vom Smp. 102-104°; $[\alpha]_D^{20} = 0 \pm 1°$ (0,5 % in $CHCl_3$); IR-Spektrum (in $CH_2Cl_2$): Absorptionsbanden bei 3420, 1740, 1710, 1675, 1600, 1575 $cm^{-1}$.

Beispiel 22: Eine Lösung von 0,310 g (0,615 mM) 4-((3R)-3-tert.Butoxycarbonylamino-3-benzhydryloxycarbonylpropoxy)-phenylmethylketon in 3 ml Pyridin wird mit 0,204 g (1,84 mM) Selendioxid versetzt. Man rührt das Gemisch während 16 Stunden bei 80°, kühlt es auf Raumtemperatur, filtriert es und dampft das klare Filtrat ein. Den Rückstand schlämmt man in 10 ml Wasser auf und stellt den pH unter Eiskühlung auf 2,5 ein. Anschliessend extrahiert man je zweimal mit 50 ml Essigester. Die organische Phase wäscht man mit Wasser, trocknet sie über Magnesiumsulfat und dampft sie ein. Man erhält die rohe 4-((3R)-3-tert.-Butoxycarbonylamino-3-benzhydryloxycarbonylpropoxy)-phenylglyoxylsäure.

Patentansprüche

1. Methodikverfahren zur Herstellung von 3-Amino-2-oxoazetidinverbindungen der Formel

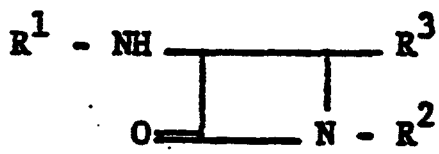

(I) ,

worin $R^1$ Wasserstoff oder eine verätherte Hydroxycarbonylgruppe der Formel $R_a^1$-O-C(=O)-, worin $R_a^1$ ein gegebenenfalls substituierter Kohlenwasserstoffrest ist, $R^2$ Wasserstoff oder einen gegebenenfalls substituierten Kohlenwasserstoffrest und $R^3$ Wasserstoff oder einen gegebenenfalls substituierten Kohlenwasserstoffrest bedeuten, worin funktionelle Gruppen gegebenenfalls geschützt sind, oder Salzen von solchen Verbindungen mit salzbildenden Gruppen, und/oder von Nitrilen der Formel $R^o$-CN, worin $R^o$ ein gegebenenfalls substituierter Phenyl- oder Heterocyclylrest ist, oder Salzen von solchen Verbindungen mit salzbildender Gruppe, dadurch gekennzeichnet, dass man eine 3-(2-Hydroxyiminoacetylamino)-2-oxoazetidinverbindung der Formel

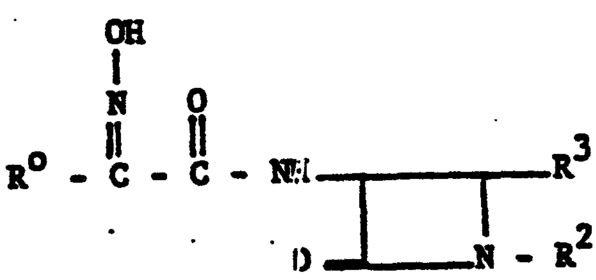

(II) ,

worin $R^o$, $R^2$ und $R^3$ die oben angegebenen Bedeutungen haben, in welchen Resten funktionelle Gruppen gegebenenfalls geschützt sind, oder ein Salz einer solchen Verbindung mit salzbildender Gruppe, in einem wasserhaltigen Medium in Gegenwart einer Base mit einem reaktionsfähigen Derivat eines Kohlensäurehalbesters der Formel $R^1_a$-O-C(=O)-OH, worin $R^1_a$ die oben angegebene Bedeutung hat, behandelt, eine Verbindung der Formel I und/oder ein Nitril $R^o$-CN isoliert und gewünschtenfalls in einer erhaltenen Verbindung gegebenenfalls vorhandene geschützte funktionelle Gruppen durch Abspaltung der Schutzgruppen freisetzt oder gegebenenfalls vorhandene freie funktionellen Gruppen schützt, und gewünschtenfalls eine erhaltene Verbindung mit salzbildender Gruppe in ein Salz oder ein erhaltenes Salz in eine freie Verbindung überführt.

2. Verfahren nach Patentanspruch 1, dadurch gekennzeichnet, dass man Verbindungen der Formel I herstellt, worin $R^1$ Wasserstoff oder eine Gruppe der Formel $R^1_a$-O-C(=O)- bedeutet, worin $R^1_a$ Niederalkyl, Oxoniederalkyl, Halogenniederalkyl, Cyanniederalkyl, Cycloalkyl, gegebenenfalls durch Halogen, Niederalkoxy oder Nitro substituiertes Phenyl, oder gegebenenfalls im aromatischen Rest durch Halogen, Niederalkoxy oder Nitro substituiertes Benzyl oder Diphenylmethyl ist.

3. Verfahren nach einem der Patentansprüche 1 oder 2, dadurch gekennzeichnet, dass man Verbindungen der Formel I herstellt, worin $R^1$ Wasserstoff oder eine Gruppe der Formel $R^1_a$-O-C(=O)- bedeutet, worin $R^1_a$ tert.-Butyloxycarbonyl oder Benzyloxycarbonyl ist.

4. Verfahren nach Patentanspruch 1, dadurch gekennzeichnet, dass man Verbindungen der Formel I herstellt, worin $R^2$ Wasserstoff, oder eine Gruppe $-CH(R^2_a)(R^2_b)$ oder eine Gruppe $-C(R^2_c)=C(R^2_d)(R^2_e)$ ist, worin $R^2_a$ freies, verestertes, amidiertes oder in Salzform vorliegendes Carboxy oder Carboxyniederalkyl, Cyan, gegebenenfalls substituiertes Amino oder gegebenenfalls substituiertes Hydroxy, $R^2_b$ gegebenenfalls, z.B. durch Niederalkyl, gegebenenfalls substituiertes Hydroxy, gegebenenfalls substituiertes Amino, Nitro oder Halogen, substituiertes Niederalkyl, Niederalkenyl, Cycloalkyl, Aryl, Heterocyclyl, Aralkyl oder Arylthioalkyl, $R^2_c$ freies, verestertes amidiertes oder in Salzform vorliegendes Carboxyl, $R^2_d$ Wasserstoff oder Niederalkyl, und $R^2_e$ Niederalkyl, gegebenenfalls, z.B. durch Niederalkyl, gegebenenfalls substituiertes Hydroxy, gegebenenfalls substituiertes Amino, Nitro oder Halogen, substituiertes Aryl, Heterocyclylthioalkyl oder Arylthio bedeutet.

5. Verfahren nach einem der Patentansprüche 1 oder 2, dadurch gekennzeichnet, dass man Verbindungen der Formel I herstellt, worin $R^2$ α-Carboxy-4-hydroxybenzyl, α-Carboxy-4-benzyloxycarbonyloxybenzyl, α-Carboxy-4-tert.-butyloxycarbonyloxybenzyl, α-Diphenylmethoxycarbonyl-4-hydroxybenzyl, α-Diphenylmethoxycarbonyl-4-tert.-butyloxybenzyl oder α-Diphenylmethoxy-4-benzyloxycarbonyloxybenzyl, bedeutet.

6. Verfahren nach Patentanspruch 1, dadurch gekennzeichnet, dass man Verbindungen der Formel I herstellt, worin $R^3$ Wasserstoff, gegebenenfalls geschütztes Hydroxymethyl, Aryl oder Aralkenyl bedeutet.

7. Verfahren nach einem der Patentansprüche 1 oder 6, dadurch gekennzeichnet, dass man Verbindungen der Formel I herstellt, worin $R^3$ Wasserstoff ist.

8. Verfahren nach Patentanspruch 1, dadurch gekennzeichnet, dass man die (3S)-3-tert.-Butyloxycarbonylamino-(αR)-α-(4-hydroxyphenyl)-2-oxo-1-azetidinessigsäure herstellt.

9. Verfahren nach Patentanspruch 1, dadurch gekennzeichnet, dass man den (3S)-3-tert.-Butyloxycarbonylamino-(αR)-α-(4-hydroxyphenyl)-2-oxo-1-azetidinessigsäure-diphenylmethylester herstellt.

10. Verfahren nach Patentanspruch 1, dadurch gekennzeichnet,dass man die (3S)-3-tert.-Butyloxycarbonylamino-(αR)-α-(4-tert.-butyloxycarbonyloxyphenyl)-2-oxo-1-azetidinessigsäure herstellt.

11. Verfahren nach Patentanspruch 1, dadurch gekennzeichnet,dass man den (3S)-3-tert.-Butyloxycarbonylamino-(αR)-α-(4-tert.-butyloxycarbonyloxyphenyl)-2-oxo-1-azetidinessigsäure-diphenylmethylester herstellt.

12. Verfahren nach Patentanspruch 1, dadurch gekennzeichnet, dass man den (3S)-3-Amino-(αR)-α-(4-tert.-butyloxycarbonyloxyphenyl)-2-oxo-1-azetidinessigsäure-diphenylmethylester, oder ein Salz davon herstellt.

13. Verfahren nach Patentanspruch 1, dadurch gekennzeichnet, dass man ein Nitril der Formel R°-CN herstellt, worin R° 4-(3-Carboxy-3-aminopropoxy)phenyl bedeutet, worin Carboxy gegebenenfalls in veresterter Form, z.B. als physiologisch spaltbare Estergruppe, wie Niederalkanoyloxymethoxycarbonyl, wie Pivaloyloxymethoxycarbonyl, oder als leicht

spaltbare Estergruppe, wie als 2,2,2-Trichloräthoxy-, Benzyloxy-, 4-Methoxybenzyloxy-, 2- oder 4-Nitrophenyloxy- oder Diphenylmethoxycarbonyl, und Amino gegebenenfalls in acylierter Form, z.B. als gegebenenfalls substituiertes Niederalkanoylamino, wie Acetylamino, 2-Chloracetylamino, 2,2,2-Trifluoracetylamino, 2-(4-Chlor-2-nitrophenoxy)-acetylamino, 2-Phenyl-2-sulfoacetylamino, Glycylamino, N-Phthaloylglycylamino, 2-Phenylglycylamino, Phthalimido, Benzoylamino, gegebenenfalls substituiertes Niederalkoxycarbonylamino oder Niederalkoxythiocarbonylamino, wie tert.-Butyloxycarbonylamino, 2-Chloräthoxycarbonylamino, 2,2,2-Trichloräthoxycarbonylamino, 4-Methoxybenzyloxy-carbonylamino, Diphenylmethoxycarbonylamino, oder Aethoxy-thiocarbonylamino, als gegebenenfalls substituiertes Ureido oder Thioureido, wie 3-Phenylureido oder 3-Phenylthioureido, oder als gegebenenfalls substituiertes Niederalkyl- oder Niederalkenylamino, wie Dimethylamino, Isopropylamino, 2-Carbomethoxyäthylamino, 2-Carboxyäthylamino, Sulfomethylamino, 1-Sulfoäthylamino, Tritylamino oder 2-Carbomethoxy-1-methylvinylamino, oder als substituiertes Arylamino, wie 2-Nitro-4-carbomethoxy-anilino, vorliegen, und worin die Phenylgruppe gegebenenfalls durch Halogen, wie Chlor, substituiert ist.

14. Verfahren nach Patentanspruch 1, dadurch gekennzeichnet, dass man als reaktionsfähiges Derivat eines Kohlensäurehalbesters der Formel $R_a^1$-O-C(=O)-OH, ein Anhydrid, ein gemischtes Anhydrid oder einen reaktionsfähigen Ester davon verwendet.

15. Verfahren nach einem der Patentansprüche 1 oder 14, dadurch gekennzeichnet, dass man das reaktionsfähige Derivat eines Kohlensäurehalbesters der Formel $R_a^1$-O-C(=O)-OH zwischen Raumtemperatur und etwa 100° zufügt und die Fragmentierung zwischen 40 und 80° erfolgen lässt.

16. Verfahren nach einem der Patentansprüche 1-15, dadurch gekennzeichnet, dass man als Ausgangsmaterial der Formel II das Nocardicin A einsetzt.

17. Verfahren nach einem der Patentansprüche 1-16, dadurch gekennzeichnet, dass man Nocardicin A mit Di-tert.-butyldicarbonat und/oder mit Benzyloxycarbonylchlorid umsetzt, und eine erhaltene Verbindung der Formel I, oder ein Salz davon, und/oder ein erhaltenes Nitril der Formel R°-CN, oder ein Salz davon, isoliert.

18. Die neuen 3-Amino-2-oxoazetidinverbindungen der Formel

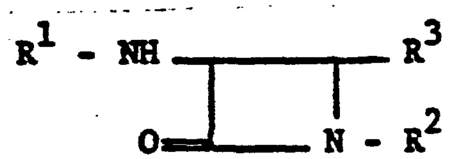

(I) ,

worin $R^1$ Wasserstoff oder eine verätherte Hydroxycarbonylgruppe der Formel $R_a^1$-O-C(=O)-, worin $R_a^1$ ein gegebenenfalls substituierter Kohlenwasserstoffrest ist, $R^2$ Wasserstoff oder einen gegebenenfalls substituierten Kohlenwasserstoffrest und $R^3$ Wasserstoff oder einen gegebenenfalls substituierten Kohlenwasserstoffrest bedeuten, worin funktionelle Gruppen gegebenenfalls geschützt sind, und Salze von solchen Verbindungen mit salzbildenden Gruppen.

19. (3S)-3-tert.-Butyloxycarbonylamino-(αR)-α-(4-hydroxyphenyl)-2-oxo-1-azetidinessigsäure gemäss Patentanspruch 18.

20. (3S)-3-tert.-Butyloxycarbonylamino-(αR)-α-(4-hydroxyphenyl)-2-oxo-1-azetidinessigsäure-diphenylmethylester gemäss Patentanspruch 18.

21. (3S)-3-tert.-Butyloxycarbonylamino-(αR)-α-(4-tert.-butyloxycarbonyloxyphenyl)-2-oxo-1-azetidinessigsäure gemäss Patentanspruch 18.

22. (3S)-3-tert.-Butyloxycarbonylamino-(αR)-α-(4-tert.-butyloxycarbonyloxyphenyl)-2-oxo-1-azetidinessigsäure-diphenylmethylester gemäss Patentanspruch 18.

23. (3S)-3-Amino-(αR)-α-(4-tert.-butyloxycarbonyloxyphenyl)-2-oxo-1-azetidinessigsäure-diphenylmethylester, oder ein Salz davon, gemäss Patentanspruch 18.

24. 3-Amino-2-oxo-azetidinverbindungen der Formel

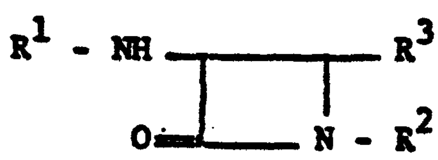

(I) ,

worin $R^1$ Wasserstoff oder eine verätherte Hydroxycarbonylgruppe der Formel $R^1_a$-O-C(=O)-, worin $R^1_a$ ein gegebenenfalls substituierter Kohlenwasserstoffrest ist, $R^2$ Wasserstoff oder einen gegebenenfalls substituierten Kohlenwasserstoffrest und $R^3$ Wasserstoff oder einen gegebenenfalls substituierten Kohlenwasserstoffrest bedeuten, worin funktionelle Gruppen gegebenenfalls geschützt sind, und Salze von solchen Verbindungen mit salzbildenden Gruppen, sofern sie

nach einem der in den Patentansprüchen 1-17 beschriebenen Verfahren hergestellt wurden.

25. Die neuen Nitrile der Formel R°-CN, worin R° ein gegebenenfalls substituierter Phenyl- oder Heterocyclylrest ist und Salze von solchen Verbindungen mit salzbildenden Gruppen.

26. Nitrile der Formel R°-CN, worin R° ein gegebenenfalls substituierter Phenyl- oder Heterocyclylrest ist und Salze von solchen Verbindungen mit salzbildenden Gruppen, sofern sie nach einem der in den Patentansprüchen 1 und 13 bis 17 beschriebenen Verfahren hergestellt wurden.

0005507

Europäisches Patentamt

# EUROPÄISCHER RECHERCHENBERICHT

Nummer der Anmeldung
EP 79 10 1415

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 529 941 (FUJISAWA)<br>* Seiten 308-317; Ansprüche *<br>-- | 1,18 |
| | DE - A - 2 645 085 (FUJISAWA)<br>* Ansprüche *<br>-- | 1,18 |
| D | DE - A - 2 714 628 (FUJISAWA)<br>* Ansprüche *<br>-- | 1,18, 25 |
| | CHEMICAL ABSTRACTS, Vol. 86, Nr. 21, 23. Mai 1977, Columbus, Ohio, USA, KUNUGITA, KIYOHIKO et al.: "3-Amino-1-(α-carboxy-4-hydroxybenzyl)-2-azetidinone", Seite 339, rechte Spalte, Zusammenfassung Nr. 153976y<br>& JP - A - 76 133 487 (FUJISAWA)<br>* Zusammenfassung *<br>-- | 1,18 |
| | JOURNAL OF THE AMERICAN CHEMICAL SOCIETY, Vol. 100, Nr. 12, 7. Juni 1978, "Total synthesis of Nocardicin A. Synthesis of 3-ANA and Nocardicin A", Seite 3933-5<br>* u.a. Seite 3934; Formeln 17,18 *<br>-- | 1,18 |
| | SYNTHESIS, Nr. 7, Juli 1977, "A convenient route to Dethio-6-aminopenicillanic acid", Seite 494-5<br>* u.a. Seite 494, Formeln 4,5 *<br>--./. | 1,18 |

KLASSIFIKATION DER ANMELDUNG (Int.Cl.²)
C 07 D 205/08
C 07 C 121/75//
C 07 C 125/06

RECHERCHIERTE SACHGEBIETE (Int. Cl.²)
C 07 D 205/08
C 07 C 121/75

KATEGORIE DER GENANNTEN DOKUMENTE
X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 08-08-1979 | CHOULY |

EPA form 1503.1 06.78

Europäisches Patentamt

# EUROPÄISCHER RECHERCHENBERICHT

Nummer der Anmeldung
EP 79 10 1415
-2-

| EINSCHLÄGIGE DOKUMENTE | | | KLASSIFIKATION DER ANMELDUNG (Int.Cl.²) |
|---|---|---|---|
| Kategorie | Kennzeichnung des Dokuments mit Angabe, soweit erforderlich, der maßgeblichen Teile | betrifft Anspruch | |
| A | DE - A - 2 657 079 (FUJISAWA)<br>* Ansprüche *<br>-- | 1,18 | |
| A | DE - A - 2 122 747 (ALLEN AND HANBURYS)<br>* Ansprüche *<br>-- | 1,18 | |
| A | FR - A - 2 102 736 (RHONE-POULENC)<br>* Ansprüche *<br>---- | 1,25 | RECHERCHIERTE SACHGEBIETE (Int. Cl.²) |